(19) Europäisches Patentamt / European Patent Office / Office européen des brevets

(11) **EP 2 676 605 B1**

(12) **EUROPEAN PATENT SPECIFICATION**

(45) Date of publication and mention
of the grant of the patent:
**05.12.2018 Bulletin 2018/49**

(51) Int Cl.:
*A61B 5/11* *(2006.01)* *A61B 5/22* *(2006.01)*
*G06Q 10/10* *(2012.01)* *G06Q 50/22* *(2018.01)*
*A61B 5/053* *(2006.01)*

(21) Application number: **11858710.4**

(22) Date of filing: **17.02.2011**

(86) International application number:
**PCT/JP2011/053408**

(87) International publication number:
**WO 2012/111132 (23.08.2012 Gazette 2012/34)**

(54) **ELECTRONIC DEVICE, PROGRAM, SERVER DEVICE, INFORMATION SYSTEM, AND HEALTH MANAGEMENT ASSISTANCE METHOD**

ELEKTRONISCHE VORRICHTUNG, PROGRAMM, SERVERVORRICHTUNG, INFORMATIONSSYSTEM UND VERFAHREN ZUR GESUNDHEITSMANAGEMENTUNTERSTÜTZUNG

DISPOSITIF ÉLECTRONIQUE, PROGRAMME, DISPOSITIF SERVEUR, SYSTÈME D'INFORMATIONS, ET PROCÉDÉ D'AIDE À LA PRISE EN CHARGE DE LA SANTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**25.12.2013 Bulletin 2013/52**

(73) Proprietor: **Fujitsu Limited**
**Kawasaki-shi, Kanagawa 211-8588 (JP)**

(72) Inventor: **NISHIDA, Asako**
**Kawasaki-shi, Kanagawa 211-8588 (JP)**

(74) Representative: **Haseltine Lake LLP**
**Lincoln House, 5th Floor**
**300 High Holborn**
**London WC1V 7JH (GB)**

(56) References cited:
**WO-A1-2007/138822 WO-A1-2010/038332**
**WO-A2-2009/131664 JP-A- 4 135 541**
**JP-A- 2001 321 372 JP-A- 2004 227 522**
**US-A1- 2010 079 291**

EP 2 676 605 B1

**Description**

FIELD

The embodiments and examples described herein relate to a device and a method for supporting health management of users.

BACKGROUND

As a method for supporting a person who implements health management (hereinafter, referred to as the "user"), the amount of activity of the user is measured, and life style improvement advice is given to the user according to the amount of activity of the user.

For example, there has been known an exercise state sensing system which includes an external arithmetic device and a user terminal which is carried by a person subject to monitoring.

In the exercise state sensing system, the user terminal includes a body motion sensing means which is attached to the monitoring subject and senses the body process state of the monitoring subject by a body motion, input means which inputs energy intake data, computing means which includes a sensing information processing function which calculates the amount of activity of the monitoring subject based on the body motion information sensed by the body motion sensing means and an energy balance calculating function which calculates an energy balance from consumption energy of the monitoring subject during a predetermined time period calculated based on basal metabolic rate per unit time of the monitoring subject and the transition data of the amount of activity during the predetermined time period and the total amount of energy intake input during the predetermined time period, storing means which temporarily stores data, display means, and communication means which establishes communications for transmitting and receiving data.

The external arithmetic device has other communication means which transmits and receives data to and from the above communication means, computing means which includes a life pattern determining function which determines the life pattern of the monitoring subject based on the transition data of the amounts of activities which are calculated by the sensing information processing means and transmitted from the user terminal and a life pattern which is registered in advance and serves as a reference and an advice function which predicts a life pattern from the present time until a predetermined time after based on the life pattern determined by the life pattern determining function and creates advice data for a better life pattern of the monitoring subject based on the predicted life pattern and the energy balance result transmitted from the user terminal, and accumulation means which accumulates the transition data of the amounts of activities and the life pattern data, the total amount data of energy intake which is input via the user terminal and the total amount data of consumption energy, the transition data of the amounts of activities, a life pattern data, and advice data.

Then, the user terminal displays the advice, based on the energy balance result and the advice data received from the computing device, on the display means.

PRIOR ART DOCUMENTS

Patent Documents

Patent document 1: Japanese Laid-open Patent Publication No. 2005-205167

SUMMARY

Characteristics, such as a user's weight, girth, and the like, are used as indexes indicative of the health degree of the user. In the following description, physical characteristics of a user, such as the user's weight, girth, and the like, are referred to as "physical characteristics".

For example, suppose that weight is used as the physical characteristics. In such a case, lowering the sum of consumption energy consumed by activities of the user and the basal metabolic rate than calorie intake increases the weight of the user. Increasing the sum of consumption energy and the basal metabolic rate than calorie intake decreases the weight of the user.

However, when a user carries out exercise of a given amount of activity, how much the physical characteristics will change according to the amount of activity involves various factors. For this reason, it was difficult to determine how much the measured amount of activity has achieved the targeting degree of improvement of the physical characteristics.

US 2010/0079291 A1 discloses a weight management system comprised of a body worn device which interfaces periodically with a computer. Weight goals of the user are translated by the computer into daily activity targets and downloaded into the device. The device monitors the user's activity, offering progress status toward the daily activity target. The computer provides historical tracking of activity for motivational and coaching purposes. The user can label time periods with an activity that they were performing during that time period, allowing activities to be recommended for reaching the user's weight goal based on the user's history.

WO 2007/138822 A1 discloses an exercise plan proposal apparatus provided with an exercise plan generation means which calculates the exercise strength and exercise time necessary to achieve an improvement target based on a set exercise operation period and the improvement target, and an exercise plan output means which presents to a user the calculated exercise strength and exercise time.

It is an object of the device and the method disclosed herein to more easily determine how much the exercise of a certain amount of activity carried out by a user achieves the target improvement of the physical characteristics.

According to an aspect of a device, there is provided an electronic device including a measurement unit which measures an amount of activity of a user according to a signal which indicates an exercise amount and is output from a sensor which detects a physical activity of the user, a required activity amount acquisition unit which acquires a required amount of activity as the amount of activity for realizing physical characteristics designated by the user, the required amount of activity being calculated by a predetermined calculation formula according to an index value of the designated physical characteristics, and a determination acquisition unit which acquires a determination of a degree of target achievement, with reference to a target amount of activity, achieved by a measured amount of activity which is measured by the measurement unit during a second measurement term after a first measurement term, the target amount of activity being determined by taking a reference amount of activity calculated based on the amount of activity measured by the measurement unit during the first measurement term into consideration with the required amount of activity;

a schedule information access unit (117) which accesses schedule information which indicates an activity plan of the user whose amount of activity is to be measured by the measurement unit (113); and

a selection unit (118) which selects, based on the schedule information, whether to include the amount of activity measured by the measurement unit (113) during a term corresponding to the schedule information in the amount of activity which is referred to when calculating the reference amount of activity.

According to an aspect of a computer program, there is provided a program which causes a computer to execute processes of measuring an amount of activity of a user according to a signal which indicates an exercise amount and is output from a sensor which detects a physical activity of the user during a first measurement term and a second measurement term after the first measurement term, acquiring a required amount of activity as the amount of activity for realizing physical characteristics designated by the user, the required amount of activity being calculated by a predetermined calculation formula according to an index value of the designated physical characteristics, acquiring a determination of a degree of target achievement, with reference to a target amount of activity, achieved by the amount of activity which is measured during the second measurement term, the target amount of activity being determined by taking a reference amount of activity calculated based on the amount of activity measured by the measurement unit during the first measurement term into consideration with the required amount of activity; accessing schedule information which indicates an activity plan of the user whose amount of activity is to be measured; and selecting, based on the schedule information, whether to include the amount of activity measured during a term corresponding to the schedule information in the amount of activity which is referred to when calculating the reference amount of activity.

Other aspects of the present invention include an information system and a health management support management method as defined in the independent claims.

Optional features of the above aspects of the present invention are defined in the dependent claims.

According to the device, system, program and method disclosed herein, it makes it possible to more easily determine how much the exercise of a certain amount of activity carried out by a user achieves the target improvement of the physical characteristics.

The object and advantages of the invention will be realized and attained by means of the elements and combinations particularly pointed out in the claims. It is to be understood that both the foregoing general description and the following detailed description are exemplary and explanatory and are not restrictive of the invention, as claimed.

BRIEF DESCRIPTION OF THE DRAWINGS

FIG. 1 is a diagram illustrating the hardware configuration of a first example of the health preservation system.
FIG. 2 is a schematic configuration view of a second example of the health preservation system.
FIG. 3 is an explanatory view of an example of processing executed by the health preservation system.
FIG. 4 is an explanatory view of a first example of processing executed in a first activity mode.
FIG. 5 is an explanatory view of a first example of processing executed in a second activity mode.
FIG. 6 is an explanatory view of a second example of processing executed in the second activity mode.
FIG. 7 is an explanatory view illustrating the state transition of the input user interface of an amount of activity.

FIG. 8 is a diagram illustrating an example of a top screen.

FIG. 9A is a diagram illustrating an example of the activity type designation screen.

FIG. 9B is a diagram illustrating an example of the activity time designation screen.

FIG. 10 is a diagram illustrating an example of the activity type registration screen.

FIG. 11 is a diagram illustrating the hardware configuration of the second example of the health preservation system.

FIG. 12 is a schematic configuration view of the second example of the health preservation system.

FIG. 13 is an explanatory view of an example of the schedule information.

FIG. 14 is an explanatory view of the second example of processing executed in the first activity mode.

FIG. 15 is a diagram illustrating the hardware configuration of a third example of the health preservation system.

FIG. 16 is a schematic configuration view of the third example of the health preservation system.

FIG. 17 is a diagram illustrating an example of the activity amount history screen.

DESCRIPTION OF EMBODIMENTS

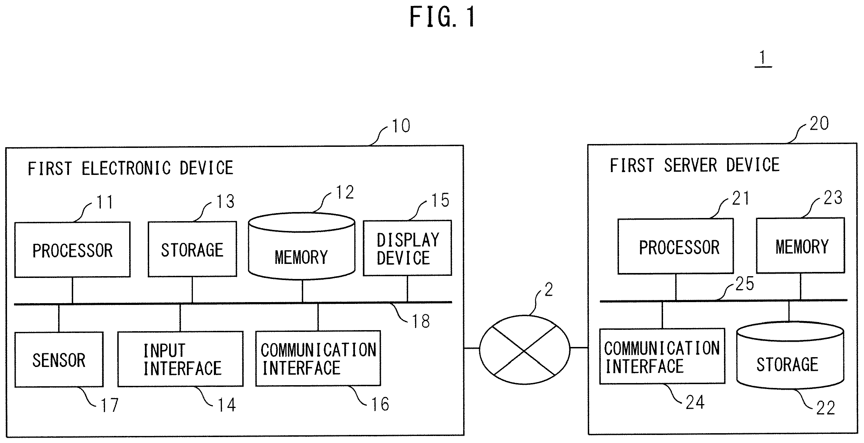

The following will describe preferred embodiments with reference to the accompanying drawings. FIG. 1 is a diagram illustrating the hardware configuration of the first example of the health preservation system. The health preservation system 1 includes a first electronic device 10 and a first server device 20 which communicate via a communication network 2. The communication network 2 may be a communication network using a wired communication line, a communication network using a wireless communication line, or a combination thereof. Note that the hardware configuration depicted in FIG. 1 is only one of the hardware configurations for implementing the health preservation system 1. Any other hardware configuration may be employed to execute the processing described hereinafter. The same also applies to the hardware configurations which will be exemplified in other embodiments.

The first electronic device 10 includes a processor 11, a storage 12, a memory 13, an input interface 14, a display device 15, a communication interface 16, a sensor 17, and a data bus 18. The first electronic device 10 is an information processing device which can communicate information with the first server device 20 via the communication network 2, and process information to be transmitted to the first server device 20 and information received from the first server device 20. The first electronic device 10 may be for example, a mobile telephone, a mobile information terminal, a personal computer, or the like.

By executing the program stored in the storage 12, the processor 11 carries out processing for controlling the operation of the first electronic device 10, and the processing described in the following for implementing the functions of the first electronic device 10 in the health preservation system 1. The storage 12 stores the program for causing the processor 11 to carry out the above processing. The storage 12 may include a hard disk, a non-volatile memory, or the like, as storing means.

The memory 13 stores a program being run by the processor 11, and data which is temporarily used by this program. The memory 13 may include a Read Only Memory (ROM) and a Random Access Memory (RAM).

The input interface 14 is an input device for accepting an input operation from a user. The input interface 14 may be, for example, a keypad, a keyboard, a pointing device, a touch panel, or the like. The display device 15 is a display device which visually displays the information processed by the first electronic device 10. The display device 15 may be, for example, a liquid crystal display, a Cathode Ray Tube (CRT) display, or an organic electroluminescence display. The communication interface 16 transmits and receives signals to and from the first server device 20. The sensor 17 senses the physical activity of a user. In some embodiments, the sensor 17 may be a vibration sensor. In another embodiment, the sensor 17 may be a triaxial acceleration sensor. The above-described constituents 11 to 17 are electrically connected by the data bus 18.

The first server device 20 includes a processor 21, a storage 22, a memory 23, a communication interface 24, and a data bus 25. By executing the program stored in the storage 22, the processor 21 carries out processing for controlling the operation of the first server device 20, and the processing described in the following for implementing the functions of the first server device 20 in the health preservation system 1. The storage 22 stores the program for causing the processor 21 to carry out the above processing. The storage 22 may include a hard disk, a non-volatile memory, or the like, as storing means.

The memory 23 stores a program being run by the processor 21, and data which is temporarily used by this program. The memory 23 may include a Read Only Memory and a Random Access Memory. The communication interface 24 transmits and receives signals to and from the first electronic device 10. The above-described constituents 21 to 24 are electrically connected by the data bus 25.

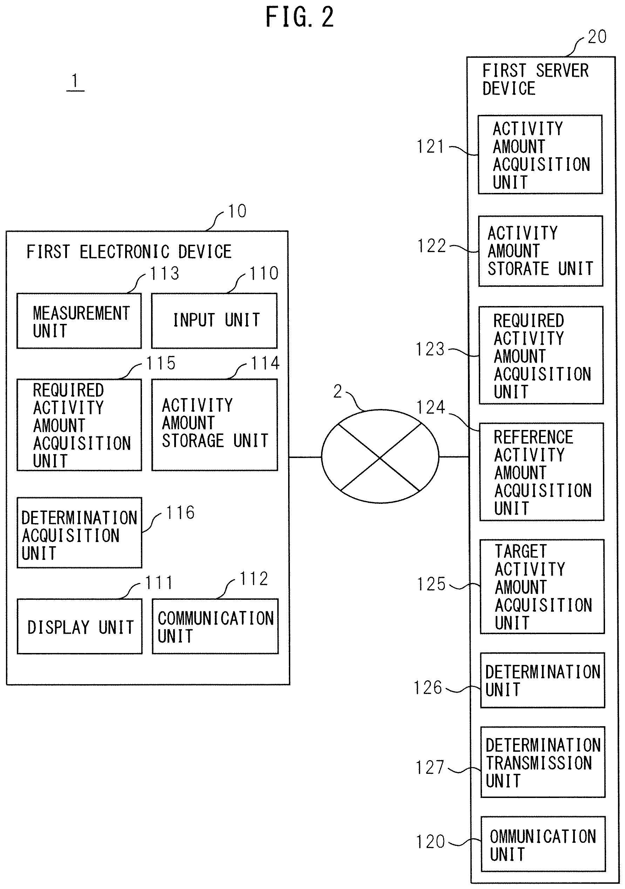

FIG. 2 is a schematic configuration view of the first example of the health preservation system. The processor 11 of FIG. 1 carries out information processing with the constituents of the first electronic device 10 depicted in FIG. 2, by following the program stored in the memory 13 and operating in coordination with the other hardware elements of the first electronic device 10 as necessary.

Similarly, the processor 21 of FIG. 1 carries out information processing with the constituents of the first server device 20 depicted in FIG. 2, by following the program stored in the memory 23 and operating in coordination with the other hardware elements of the first server device 20 as necessary. Note that FIG. 2 mainly illustrates functions related to the following description. Thus, the first electronic device 10 and the first server device 20 may also include other constituents than the constituents of FIG. 2. Note that the health preservation system 1 may be implemented as a stand-alone computer system. In such a case, the processor 11 of the first electronic device 10 of FIG. 1 may also carry out the information processing performed by the first server device 20 as will be described hereinafter.

The first electronic device 10 includes an input unit 110, a display unit 111, a communication unit 112, a measurement unit 113, an activity amount storage unit 114, a required activity amount acquisition unit 115, and a determination acquisition unit 116. The input unit 110 carries out acceptance processing of a variety of information which is input by a user via the input interface 14. In some embodiments, the input unit 110 accepts input of index values which indicate physical characteristics which a user desires.

The display unit 111 displays, through the display device 15, the input acceptance screen for accepting information input from the input unit 110. Further, the display unit 111 carries out processing of displaying information which is processed or calculated by the first electronic device 10 or information which the first electronic device 10 has received from the first server device 20. The communication unit 112 carries out communication processing for exchanging information between the first electronic device 10 and the first server device 20.

The measurement unit 113 measures amounts of activities of a user according to signals indicating the exercise amount which is output from the sensor 17. The measured amounts of activities are stored in the activity amount storage unit 114. The measured amounts of activities are transmitted to the first server device 20 via the communication unit 112. In some embodiments, the communication unit 112 may periodically transmit the measurement data of the amounts of activities stored in the activity amount storage unit 114 to the first server device 20.

The activity amount storage unit 114 may be omitted. For example, in some embodiments, the first electronic device 10 may directly transmit the measurement data of the amounts of activities to the first server device 20. The first server device 20 may store the amounts of activities received from the first server device 20 in the activity amount storage unit 122.

The required activity amount acquisition unit 115 acquires a required amount of activity which is calculated by a predetermined calculation formula according to the index value of the physical characteristics designated by a user. For example, in some embodiments, a user may designate targeting weight as the index value of the physical characteristics. In this embodiment, the required activity amount acquisition unit 115 may acquire an amount of activity of exercise which the user should do additionally to the activities of the ordinary lifestyle in order to achieve the target weight. Note that a user who designates an index value which indicates physical characteristics based on which the required amount of activity is calculated may be the same person as, for example, a user whose amount of activity is measured by the measurement unit 113, i.e., for example, a user who carries the first electronic device 10, or a different person.

The required activity amount acquisition unit 115 may calculate the required amount of activity according to the index value of the physical characteristics designated by a user. Further, in some embodiments, the first server device 20 may calculate the required amount of activity. In such a case, the required activity amount acquisition unit 115 acquires the required amount of activity which is transmitted from the first server device 20 and received by the first electronic device 10. The first electronic device 10 may transmit a variety of data used for calculating the required amount of activity to the first server device 20. In this embodiment, the first electronic device 10 calculates the required amount of activity.

A user may input the index values of physical characteristics to the first electronic device 10 via the input unit 110. In some embodiments, a user may load the index values of physical characteristics which have been input using other electronic device to the first electronic device 10 via the communication network 2.

The determination acquisition unit 116 acquires the determination of the degree of target achievement which indicates how much the amount of activity measured by the measurement unit 113 achieved a target amount of activity. The display unit 111 displays the acquired determination on the display device 15. In some embodiments, a target amount of activity is determined by taking the reference amount of activity calculated based on the amounts of activities measured by the measurement unit 113 during the first measurement term into consideration with the above-described required amount of activity.

In some embodiments, the reference amount of activity is calculated as an average amount of activity of the amounts of activities measured during the first measurement term. The first measurement term, for example, may be a term before a user starts additional exercise in addition to activities in daily lifestyle according to the health management supported by the system of this embodiment.

In the following description, a state in which a user carries out activities in daily lifestyle is referred to as the "normal time". The normal time may be, for example, a state in which a user is doing exercise of an average amount of activity during a term before the user starts exercise in addition to activities in daily lifestyle according to the health management supported by the system of this embodiment. "A state in which a user is doing exercise of an average amount of activity" is, for example, a state in which activities with calorie consumption that is significantly different from those in a daily lifestyle, such as, going on a business trip, being hospitalized, doing a workout, or the like are not performed.

The reference amount of activity may be an average amount of activity of a user in normal time. In other words, the consumption energy and energy intake of the user are assumed to be balanced when the user is performing activities of the reference amount of activity. Thus, by taking the required amount of activity into consideration with the reference amount of activity, the total amount of amounts of activities for realizing the desired physical characteristics is determined as a target amount of activity.

In some embodiments, the first electronic device 10 may calculate a reference amount of activity. In such a case, the processor 11 may carry out processing of the reference activity amount acquisition unit which calculates the reference amount of activity. In another embodiment, the first server device 20 may calculate a reference amount of activity. In such a case, the processor 11 may carry out processing of the reference activity amount acquisition unit which acquires the reference amount of activity transmitted from the first server device 20 to the first electronic device 10. The first electronic device 10 may transmit a variety of data used for calculating the reference amount of activity to the first server device 20. In this embodiment, the first server device 20 calculates the reference amount of activity.

In some embodiments, the first electronic device 10 may calculate a target amount of activity. In such a case, the processor 11 may carry out processing of the target activity amount acquisition unit which acquires a target amount of activity by calculating the target amount of activity. The communication unit 112 of the first electronic device 10 may receive the reference amount of activity calculated by the first server device 20 from the first server device 20. The communication unit 112 may also receive the required amount of activity calculated by the first server device 20 from the first server device 20.

In another embodiment, the first server device 20 may calculate a target amount of activity. In such a case, the processor 11 may carry out processing of the target activity amount acquisition unit which acquires the target amount of activity transmitted from the first server device 20 to the first electronic device 10. The first electronic device 10 may transmit a variety of data used for calculating the target amount of activity to the first server device 20. In this embodiment, the first server device 20 calculates the target amount of activity.

In some embodiments, a degree of target achievement is determined based on how far the measured amount of activity measured by the measurement unit 113 during the second measurement term after the first measurement term achieved the target amount of activity. The determination of the degree of target achievement may be a result of determination indicating whether or not the measured amount of activity during the second measurement term achieved the target amount of activity. In another embodiment, the determination of the degree of target achievement may be a difference or a rate of the measured amount of activity during the second measurement term and the target amount of activity.

In some embodiments, the determination acquisition unit 116 may acquire a message determined in accordance with the measured amount of activity and the target amount of activity during the second measurement term, as the determination of the degree of target achievement.

In another embodiment, the determination acquisition unit 116 may process determination of the degree of target achievement. The communication unit 112 of the first electronic device 10 may receive the target amount of activity calculated by the first server device 20 from the first server device 20. Further, in another embodiment, the first server device 20 may process determination of the degree of target achievement. In such a case, the required activity amount acquisition unit 115 acquires the determination which is transmitted from the first server device 20 and received by the first electronic device 10. In this embodiment, the first server device 20 processes determination of the degree of target achievement.

The following will describe a specific example of processing for calculating a required amount of activity, a reference amount of activity, and a target amount of activity. In the following processing, the weight of a user is used as an example of physical characteristics. The present weight of the user is defined as $W1$ [kg] and the target weight is defined as $W2$ [kg]. Further, a required number of days for changing the weight of the user to $W2$ [kg] is defined as $Dr$. To realize this target weight, the user needs to make energy deficiency ($Ei-Ec$) in energy intake $Ei$ in relation to consumption energy Ec during the required number of days $Dr$ the value given by the following formula (1):

$$(Ei - Ec) = (W1 - W2) \times 7000 \ [kcal] \qquad (1)$$

Energy deficiency per day $Er$ obtained by dividing energy deficiency ($Ei-Ec$) by the required number of days is given by the following formula (2):

$$Er = (W1 - W2) \times 7000/Dr \qquad (2)$$

The user can realize the target weight *W2* by increasing consumption energy every day by the amount of energy deficiency per day *Er* from normal time, or decreasing energy intake from normal time.

For example, suppose the user increases consumption energy everyday by the amount of energy deficiency per day *Er* from normal time. In such a case, the required amount of activity *Ar* is an amount of activity for consuming energy deficiency per day *Er* [EX]. The required amount of activity *Ar* is determined by the following predetermined calculation formula (3):

$$Ar = Er/W1/1.05 \qquad (3)$$

The user may lower the required amount of activity *Ar* than a value determined by the formula (3) by decreasing energy intake than normal time. The required amount of activity *Ar* in this case can be determined by the following predetermined formula (4):

$$Ar = Er \times Re/W1/1.05 \qquad (4)$$

Here, the coefficient *Re* is a rate of the increment of consumption energy in relation to the sum of the increment of consumption energy and the decrement of energy intake (Re = Increment of consumption energy / (Increment of consumption energy + Decrement of energy intake)).

The target amount of activity *At* is a target value of the total amount of activity of a user during a given term. In this embodiment, the target value of the total amount of activity per day of a user is defined as a target amount of activity *At.* The target amount of activity *At* is determined by taking a reference amount of activity Ab which is an amount of activity of the user at normal time into consideration with a required amount of activity *Ar* which is an amount of activity of exercise additionally performed to activities in daily lifestyle.

The reference amount of activity *Ab* may be an average value of amounts of activities of a user per day during the first measurement term. The target amount of activity *At* is determined by the following formula (5):

$$At = Ar + Ab \qquad (5)$$

The following will describe an example of calculation of a required amount of activity, a reference amount of activity, and a target amount of activity. The present weight *W1* of a user is defined as 85 kg, and a target weight *W2* is defined as 80 kg. The required number of days *Dr* is defined as 90 days, and the ratio of the consumption energy increment to the energy intake decrement during this term is defined as 3:2. That is, the coefficient is *Re* = 0.6. Further, the length of the first measurement term for determining the reference amount of activity *Ab* is defined as two weeks, and the reference amount of activity *Ab* is 2.3 EX. Note that the ratio of the consumption energy increment to the energy intake decrement may be changed by the user. For example, the user may designate the ratio of the consumption energy increment to the energy intake decrement via the input unit 110.

The energy deficiency per day Er is obtained by the formula (2):

$$Er = (85-80) \times 7000/90 = 389 \text{ kcal}$$

The required amount of activity *Ar* is obtained by the formula (4):

$$Ar = 389 \times 0.6/85/1.05 = 2.6 \text{ EX}$$

The target amount of activity *At* is obtained by the formula (5):

$$At = 2.6 + 2.3 = 4.9 \text{ EX}$$

Next, the following will describe one specific example of processing for determining the degree of target achievement in relation to the target amount of activity *At.* The degree of target achievement is determined by determining how much the measured amount of activity of a user measured by the measurement unit 113 during the predetermined term achieved the target amount of activity *At.* The degree of target achievement in this embodiment is determined by whether or not the measured amount of activity per day during the second measurement term after the first measurement term is equal to or more than the target amount of activity *At*.

To inform the determination of the degree of target achievement to a user, a support message is generated according to a comparison result of the measured amount of activity and the target amount of activity *At*. For example, when the measured amount of activity is equal to or more than the target amount of activity *At*, a message to commend the user is generated. When the measured amount of activity is less than the target amount of activity *At*, a message to encourage the user is generated.

Next, the configuration and processing of the first server device 20 will be described. The first server device 20 includes a communication unit 120, an activity amount acquisition unit 121, an activity amount storage unit 122, a required activity amount acquisition unit 123, a reference activity amount storage unit 124, a target activity amount storage unit 125, a determination unit 126, and a determination transmission unit 127.

The communication unit 120 carries out communication processing for exchanging information between the first electronic device 10 and the first server device 20. The activity amount acquisition unit 121 acquires the measured amount of activity of a user measured according to signals indicating an exercise amount which is output from the sensor 17 of the first electronic device 10. In some embodiments, the activity amount acquisition unit 121 acquires the measured amount of activity of a user measured by the first electronic device 10 and transmitted to the first server device 20. In another embodiment, the activity amount storage unit 122 for storing the measured amount of activity received from the first electronic device 10 may be provided.

The required activity amount acquisition unit 123 acquires a required amount of activity calculated by a predetermined calculation formula according to the index value of physical characteristics designated by a user. In some embodiments, the first electronic device 10 may calculate the required amount of activity. In such a case, the required activity amount acquisition unit 123 acquires the required amount of activity transmitted from the first electronic device 10 to the first server device 20. Further, in another embodiment, the required activity amount acquisition unit 123 may calculate the required amount of activity according to the index value of physical characteristics designated by the user. In such a case, the first server device 20 may receive a variety of data used for calculating the required amount of activity from the first electronic device 10. As described above, in this embodiment, the first electronic device 10 calculates the required amount of activity.

The reference activity amount acquisition unit 124 acquires a reference amount of activity. In some embodiments, the first electronic device 10 may calculate the reference amount of activity. In such a case, the reference activity amount acquisition unit 124 acquires the reference amount of activity transmitted from the first electronic device 10 to the first server device 20. In another embodiment, the reference activity amount acquisition unit 124 may calculate the reference amount of activity. In such a case, the first server device 20 may receive a variety of data used for calculating the reference amount of activity from the first electronic device 10. As described above, in this embodiment, the first server device 20 calculates the reference amount of activity.

The target activity amount acquisition unit 125 acquires a target amount of activity which is determined by taking the reference amount of activity into consideration with the required amount of activity. In some embodiments, the first electronic device 10 may calculate the target amount of activity. In such a case, the target activity amount acquisition unit 125 acquires the target amount of activity which is transmitted from the first electronic device 10 to the first server device 20. Further, in another embodiment, the target activity amount acquisition unit 125 may calculate the required amount of activity. In such a case, the first server device 20 may receive a variety of data used for calculating the reference amount of activity from the first electronic device 10. As described above, in this embodiment, the first server device 20 calculates the target amount of activity.

The determination unit 126 determines a degree of target achievement which indicates how much the measured amount of activity measured by the measurement unit 113 achieved the target amount of activity. The determination unit 126 may periodically determine the degree of target achievement. In some embodiments, the determination unit 126 determines the degree of target achievement in a once-a-day cycle. The determination transmission unit 127 transmits the determination by the determination unit 126 to the first electronic device 10. In another embodiment, the first electronic device 10 may carry out determination processing of a degree of achievement of the target amount of activity. In such a case, the determination unit 126 and determination transmission unit 127 may be omitted. In this embodiment, the first server device 20 determines the degree of achievement of the target amount of activity.

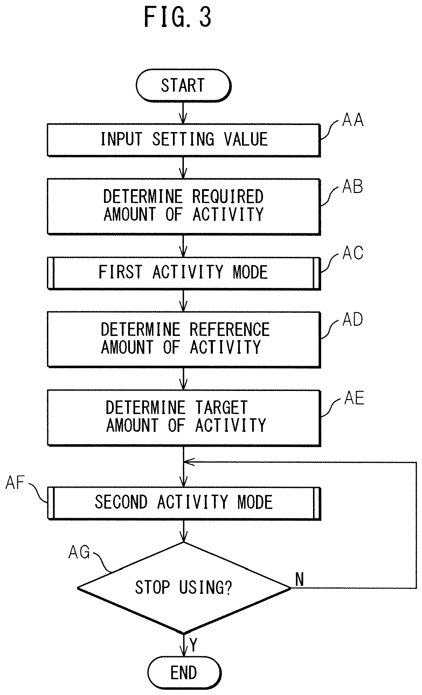

Next, with reference to FIG. 3, the processing carried out by the health preservation system 1 of this embodiment will be described. Note that in other embodiments, each operation of the following operations AA to AG may be referred to as a step.

At operation AA, the input unit 110 processes acceptance of a setting value input by a user. The setting value includes the index value of physical characteristics of the user. For example, the index value of physical characteristics includes the present weight of the user and target weight. For example, the setting value may include a required number of days for realizing the targeting physical characteristics. The setting value may include coefficient *Re* which is the ratio of the consumption energy increment to the sum of the consumption energy increment and the energy intake decrement for realizing the physical characteristics.

At operation AB, the required activity amount acquisition unit 115 of the first electronic device 10 determines a required amount of activity. The first electronic device 10 transmits the required amount of activity to the first server device 20. In the first activity mode of operation AC, the measurement unit 113 of the first electronic device 10 measures amounts of activities of the user during the first measurement term. The measurement processing of the amounts of activities of the user in the first activity mode will be described hereinafter.

When the first measurement term is expired, the reference activity amount acquisition unit 124 of the first server device 20 determines a reference amount of activity based on the amounts of activities of the user measured during the first measurement term at operation AD. At operation AE, the target activity amount acquisition unit 125 determines a target amount of activity which is determined by taking the reference amount of activity into consideration with the required amount of activity.

In the second activity mode of operation AF, the measurement unit 113 of the first electronic device 10 measures the amounts of activities of the user during the second measurement term. In the second activity mode, the determination unit 125 of the first server device 20 determines how much the measured amount of activity measured by the measurement unit 113 achieved the target amount of activity. The determination acquisition unit 116 of the first electronic device 10 acquires the determination of the degree of target achievement. In the following, the details of the processing in the second activity mode will be described.

At operation AG, the first electronic device 10 determines whether there is a termination instruction of the health preservation system from the user. When there is a termination instruction (operation AG: Y), the processing is terminated. When there is no termination instruction (operation AG: N), the processing returns to operation AF.

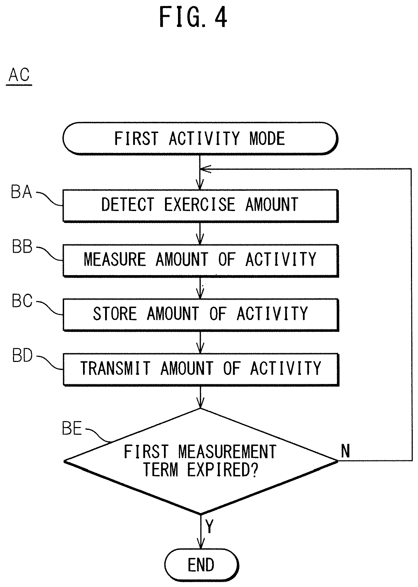

Next, with reference to FIG. 4, the first example of processing carried out in the first activity mode will be described. Note that in other embodiments, each operation of the following operations BA to BE may be referred to as a step.

At operation BA, the sensor 17 of the first electronic device 10 measures an exercise amount of a user, and outputs signals indicating the measured exercise amount. At operation BB, the measurement unit 113 measures the amounts of activities of the user according to the output signals from the sensor 17. At operation BC, the measurement unit 113 stores the measured amounts of activities in the activity amount storage unit 114. At operation BD, the first electronic device 10 transmits the amounts of activities stored in the activity amount storage unit 114 to the first server device 20.

At operation BE, the first electronic device 10 determines whether the first measurement term has expired. When the first measurement term has expired (operation BE: Y), the first electronic device 10 terminates the first activity mode. When the first measurement term has not yet expired (operation BE: N), the processing returns to operation BA.

Note that the measurement result of the amounts of activities measured by the measurement unit 113 during the first measurement term is stored in the activity amount storage unit 114 in a manner concealed from the user. For example, the measurement result of the amounts of activities may be stored in a storage area which is configured in a hardware side so that the user is not able to access through an operation on the input unit 110. Further, for example, the measurement result of the amounts of activities may be stored in the activity amount storage unit 114 after being encrypted using an encryption key which is concealed from the user. Further, for example, the first electronic device 10 may include a memory access control unit for restricting the user to refer to the amounts of activities stored in the activity amount storage unit 114 only during the first measurement term.

Further, the measurement result of the amounts of activities measured by the measurement unit 113 during the first measurement term is stored in the activity amount storage unit 122 of the first server device 20. The measured amounts of activities during the first measurement term may also be stored in the activity amount storage unit 122 in a manner concealed from the user.

As described above, the amounts of activities at normal time which are base data for determining a reference amount of activity is measured with regard to the first measurement term. It is not possible to accurately calculate the reference amount of activity when a user intends to increase the amounts of activities during the first measurement term. Thus, concealing the measurement result of the amounts of activities during first measurement term from the user prevents the user from intentionally changing the amount of activity which is being measured while being aware of the amount of activity, whereby appropriate measurement data can be obtained.

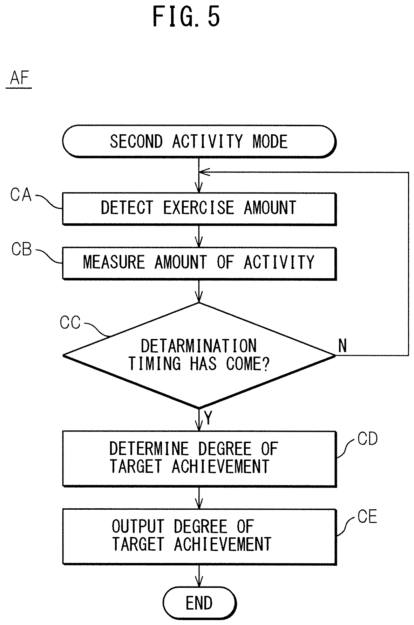

Next, with reference to FIG. 5, the first example of processing carried out in the second activity mode will be described. Note that in other embodiments, each operation of the following operations CA to CE may be referred to as a step.

At operation CA, the sensor 17 of the first electronic device 10 measures the exercise amount of a user, and outputs signals indicating the measured exercise amount. At operation CB, the measurement unit 113 measures the amount of activity of the user according to the output signals from the sensor 17. The first electronic device 10 transmits the measured amount of activity to the first server device 20.

At operation CC, the determination unit 125 of the first server device 20 determines whether determination timing has come yet for determining how much the measured amount of activity measured at operations CA and CB achieved the target amount of activity. For example, when a predetermined time of day which is set by a user in advance has come, the determination unit 125 may determine that the determination timing has come. When the determination timing has come (operation CC: Y), the processing proceeds to operation CD. When the determination timing has not come yet (operation CC: N), the processing returns to operation CA.

At operation CD, the determination unit 125 determines how much the measured amount of activity measured at operations CA and CB achieved the target amount of activity. The determination transmission unit 127 transmits the determination by the determination unit 126 to the first electronic device 10. At operation CE, the display unit of the first electronic device 10 outputs the determination to the display device 15.

The health preservation system of this embodiment measures an amount of activity of a user at normal time as a reference amount of activity of a user, and determines a target amount of activity which is the target of a total amount of activity carried out by the user by taking into consideration a required amount of activity which the user has done additionally to realize the desired physical characteristics. A user can determine the target amount of activity by inputting data for calculating the required amount of activity. Thus, according to this embodiment, the target amount of activity can be easily determined. For example, when calculating the target amount of activity using the above formulas (3) and (5), the user only has to input the present weight $W1$, the target weight $W2$, and the required number of days $Dr.$

Further, the first electronic device 10 and the first server device 20 of the health preservation system 1 of this embodiment selectively operates either in the first activity mode where a measurement value is acquired to calculate the reference amount of activity during the first measurement term, or in the second activity mode where the degree of target achievement is determined during the second measurement term. By separating a term for calculating the reference amount of activity from a term for determining the degree of target achievement, the measurement value is prevented from being affected by feeding back to the user the determination of the degree of target achievement during the term for measuring the measurement value which is a base for calculating the reference amount of activity.

Next, another example of processing of the health preservation system 1 will be described. Exercise with a high amount of activity is preferable in health management of a user, and recommended in terms of health management. For example, playing sports is preferable for health management of a user. Moreover, exercise with a high amount of activity plays a significant role for realizing the desired physical characteristics of a user.

However, a user does not necessarily carry the first electronic device 10 when carrying out an activity with high consumption energy. For example, when swimming or doing martial arts, a user might possibly take off the first electronic device 10. In such a case, the first electronic device 10 is not able to measure the amount of activity of the user, thereby the activity is not reflected to the above-described target achievement determination by the health preservation system 1. When exercise with a high amount of activity is not reflected to the determination of the degree of target achievement, the user is not given enough motivation to carry out such an exercise.

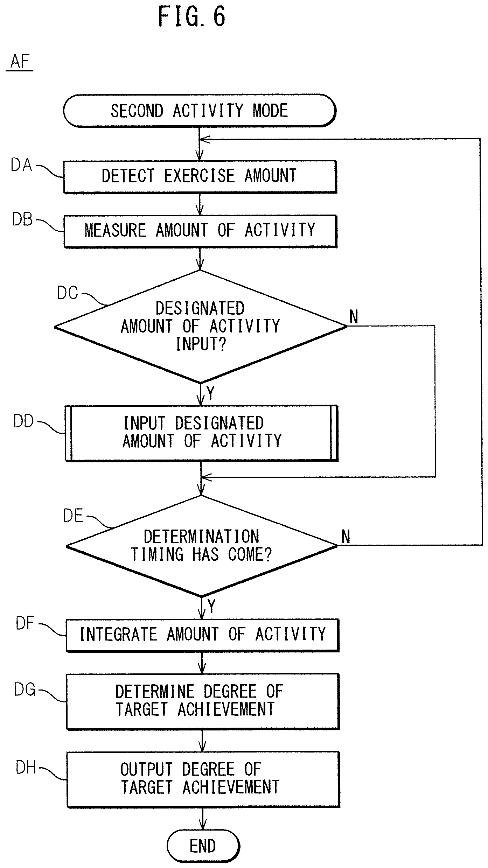

The health preservation system 1 of the following embodiment allows a user to input an amount of activity, and determines a degree of achievement of a target amount of activity based on an amount of activity which is obtained by integrating the input amount of activity with the amount of activity measured by measurement unit 113. With reference to FIG. 6, processing of the second activity mode in this embodiment will be described. In other embodiments, each operation of the following operations DA to DH referred to as a step

The processing of operations DA and DB are the same as those of operations CA and CB illustrated in FIG. 5. At operation DC, the input unit 110 determines whether there is an instruction from a user who inputs the amount of activity. When there is an instruction (operation DC: Y), the processing proceeds to operation DD. When there is no instruction (operation DC: N), the processing proceeds to operation DE. In the following description, the amount of activity input by a user may be referred to as the "designated amount of activity".

At operation DD, the display unit 111 displays an input user interface for the user to input a designated amount of activity on the display device 15. The input unit 110 accepts the designated amount of activity which the user has input by following the input user interface. The first electronic device 10 transmits the input designated amount of activity to the first server device 20. The first server device 20 stores the designated amount of activity in the activity amount storage unit 122. The input user interface generated by the display unit 111 will be described hereinafter.

At operation DE, the determination unit 125 determines whether the determination timing for determining the degree of achievement of the target amount of activity has come. When the determination timing has come (operation DE: Y), the processing proceeds to operation DF. When the determination timing has not come yet (operation DE: N), the processing returns to operation CA.

At operation DF, the determination unit 125 integrates the measured amount of activity measured at operation DA and DB and the designated amount of activity input at operation DD. At operation DG, the determination unit 125 determines how much the amount of activity obtained by integrating the measured amount of activity and the designated amount of activity has achieved the target amount of activity. The processing of operation DH is the same as operation CE illustrated in FIG. 5.

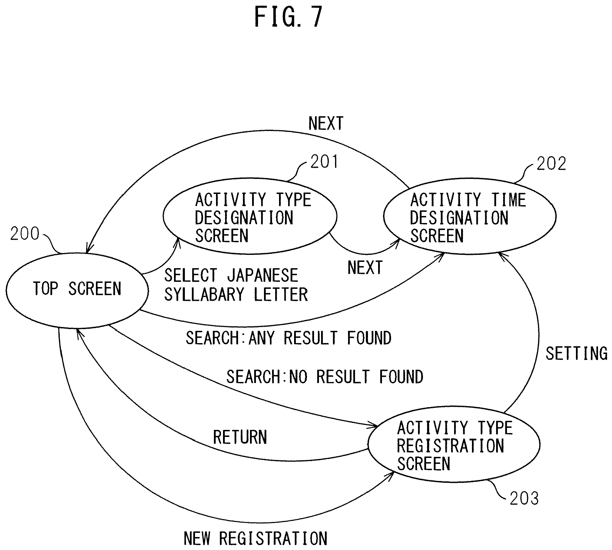

The following will describe the input user interface generated by the display unit 111. Fig. 7 is an explanatory view of state transitions of the input user interface. In states 200 to 203, a top screen, an activity type designation screen, an activity time designation screen, and an activity type registration screen, are displayed respectively. Each screen will be briefly described in the following.

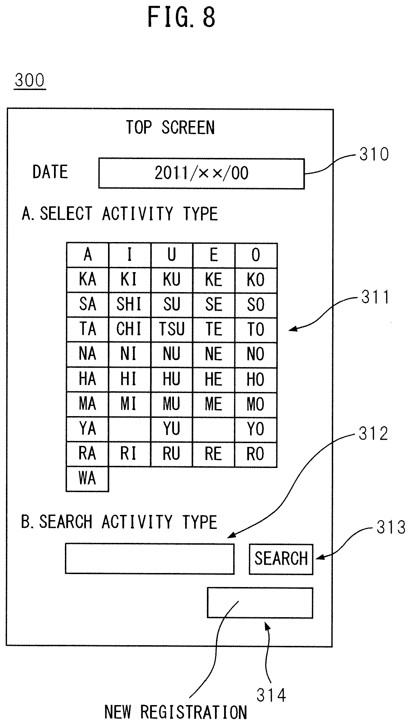

FIG. 8 is a diagram illustrating an example of the top screen displayed in state 200. The top screen 300 includes a due date input field 310, a Japanese syllabary selection field 311, a search word input field 312, a Search button 313, and a New Registration button 314. The due date input field 310 is an input object for inputting a due date for carrying out the activity of the designated amount of activity. The display unit 111 may automatically input the present due date in the due date input field 310 when displaying the top screen 300.

With the input interface as described below, a user can designate which type of activity the activity amount is input as a designated amount of activity. The first electronic device 10 stores activity intensity for each kind of activity in the storage 12. The user can designate the designated amount of activity by inputting the kind of activity and time during which the activity was being carried out. Thus, the user can designate the designated amount of activity corresponding to the activity which the user has carried out without knowing the intensity of the activity. In the following description, the type of activity is referred to as the "activity type".

The Japanese syllabary selection field 311 is an input object for selecting the initial letter of an activity type. When any one of displayed Japanese syllabary letters is selected in the Japanese syllabary selection field 311, the present state transits to state 201. In this example, a letter "Su" is selected.

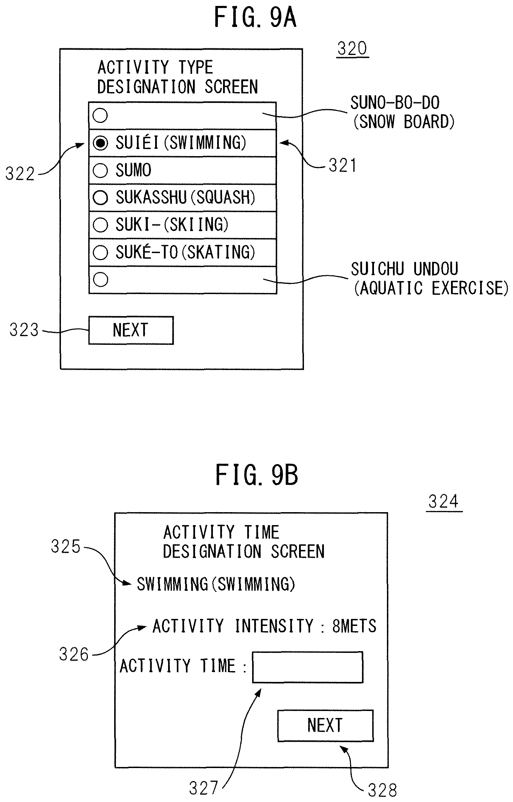

FIG. 9A illustrates an example of the activity type designation screen which is displayed in state 201. The user designates an activity type in the activity type designation screen 320. The activity type designation screen 320 includes an activity type list 321 of names starting from the letter selected at the Japanese syllabary selection field 311, radio buttons 322 provided corresponding to the display of respective activity types, and a Next button 323. In this example, the activity type list 321 includes activity types starting from "Su," "*suno-bo-do* (snowboard)," "*suiéi* (swimming)," "*sumo*," and the like.

When the user designates any one of activity types in the list 321 by the radio button 322, and presses the Next button 323, the present state transits to state 202. In this example, an activity type "*suiéi* (swimming)" is designated. FIG. 9B illustrates an example of the activity time designation screen displayed in state 202.

The user inputs a time length during which the user has carried out the designated activity type in the activity time designation screen 324. The activity time designation screen 324 includes an activity type display field 325 which displays the designated activity type, an activity intensity display field 326 which displays the activity intensity of an activity type, an activity time input field 327, and a Next button 328. The activity time input field 327 is an input object for inputting a time length during which the activity of the designated amount of activity has been carried out. The designated amount of activity to be input is designated by a value obtained by multiplying the activity intensity $I$ displayed in the activity intensity display field 326 by a time length $T$ input in the activity time input field 327 ($I \times T$). For example, in the example illustrated in FIG. 9B, when 1.5 hours is input as activity time, the designated amount of activity becomes $8 \times 1.5 = 12$ EX. When an operation of pressing the Next button is carried out, the input unit 113 generates data indicating the designated amount of activity. Then, the present state transits to state 200, and the top screen 300 is displayed again.

When the user inputs the name of an activity type in the search word input field 321 of the top screen 300, and carries out processing of pressing the Search button 313, the input unit 113 searches the activity type among the activity types registered in the storage 12. When the input activity type has been found, the present state transits to the above-described state 202. At state 202, the activity time designation screen 324 is displayed for inputting activity time for the found activity type.

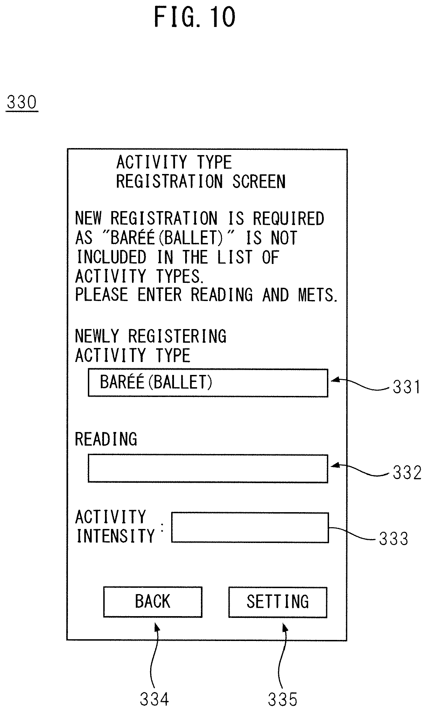

When the input activity type has not been found, the present state transits to state 203. FIG. 10 illustrates an example of the activity type registration screen which is displayed in state 203. In the activity type registration screen 330, the user can register an activity type which has not been found in the registered activity types as the result of searching and the activity intensity thereof in the storage 12. In this example, a case in which an activity type "*barée* (ballet)" is input will be described.

The activity type registration screen 330 includes an activity type input field 331, a reading field 332, an activity intensity input field 333, a Back button 334, and a Setting button 335. The activity type input field 331 is an input object for inputting an activity type of which activity intensity is to be registered in the storage 12. The display unit 111 may automatically input the activity type which has not been found by searching in the activity type input field 331 when displaying the activity type registration screen 330. The reading field 332 is an input object for inputting reading of an activity type input in the activity type input field 331.

The activity intensity input field 333 is an input object for inputting the activity intensity of the input activity type. When a user carries out an operation of pressing the Setting button 335, the input unit 113 registers the input activity type and the activity intensity thereof in the storage 12. Then, the present state transits to state 202, and the activity time designation screen 324 for inputting activity time with regard to the registered activity type is displayed. When the Back button 334 is pressed, the present state transits to state 200, and the top screen 300 is displayed again.

When the user presses the New Registration button on the top screen 300, the present state transits to 203, and the activity type registration screen 330 for newly registering the activity type and the activity intensity in the storage 12 is displayed.

According to this embodiment, the user can input the amount of activity which has been carried out when the user was not carrying the first electronic device 10 to the health preservation system 1. As a result, for example, when exercise with a relatively high amount of activity has been carried out without carrying the first electronic device 10, the amount of activity can be reflected to the determination of the degree of achievement of the target amount of activity. Therefore, in the health preservation system 1 of this embodiment, the user is encouraged to carry out exercise or a sport which is done without carrying the first electronic device 10.

Next, another embodiment of the health preservation system 1 will be described. The reference amount of activity calculated in the health preservation system 1 is an average amount of activity of a user at normal time. Therefore, measurement values at time during which calorie consumption is significantly different from those in a daily lifestyle, such as when going on a business trip, being hospitalized, and doing a workout, are preferably excluded from the measurement values of amounts of activities of the user as basis for calculating the reference amount of activity. In the health preservation system 1 of the embodiment described below, amounts of activities which are referred to when calculating the reference amount of activity are selected according to the schedule information which indicates the activity plan of the user of the first electronic device 10.

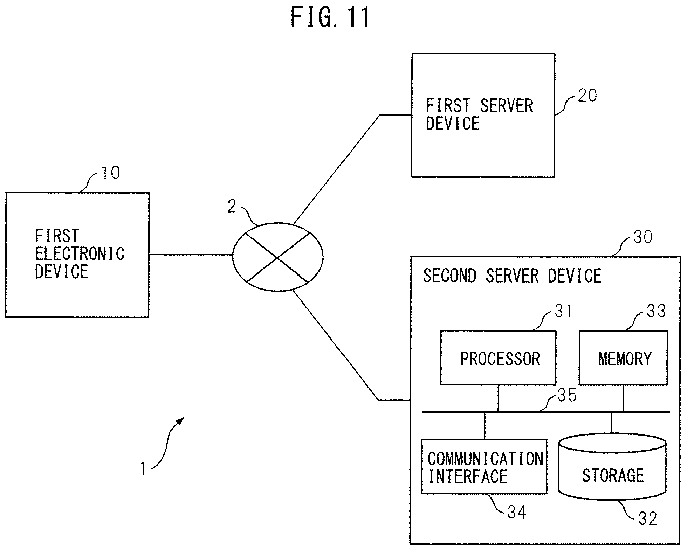

FIG. 11 is a diagram illustrating a hardware configuration of the second example of the health preservation system 1. The same reference numerals are assigned to constituents equivalent to the constituents depicted in FIG. 1. The operations of the constituents assigned the same reference numerals are the same unless otherwise particularly mentioned. Other embodiments may have the constituents depicted in FIG. 11 and other functions. The first electronic device 10 of the health preservation system 1 can access the second server device 30 via the communication network 2. The second server device 30 may be a part of the health preservation system 1, or a server device outside of the health preservation system 1.

The second server device 30 includes a processor 31, a storage 32, a memory 33, a communication interface 34, and a data bus 35. The processor 31 carries out processing for controlling the operation of the second server device 30 or the processing described hereinafter by executing a program stored in the storage 32. The storage 32 stores the program for causing the processor 31 to execute the above-described processing. The storage 32 may include a hard disk, a non-volatile memory, or the like as storing means.

The memory 33 stores a program being run by the processor 31 and data which is temporarily used by the program. The memory 33 may include a read only memory, a random access memory, or the like. The communication interface 34 carries out transmission and reception processing of signals between the first electronic device 10 and the second server device 30. The above-described constituents 31-34 are electrically connected through the data bus 35.

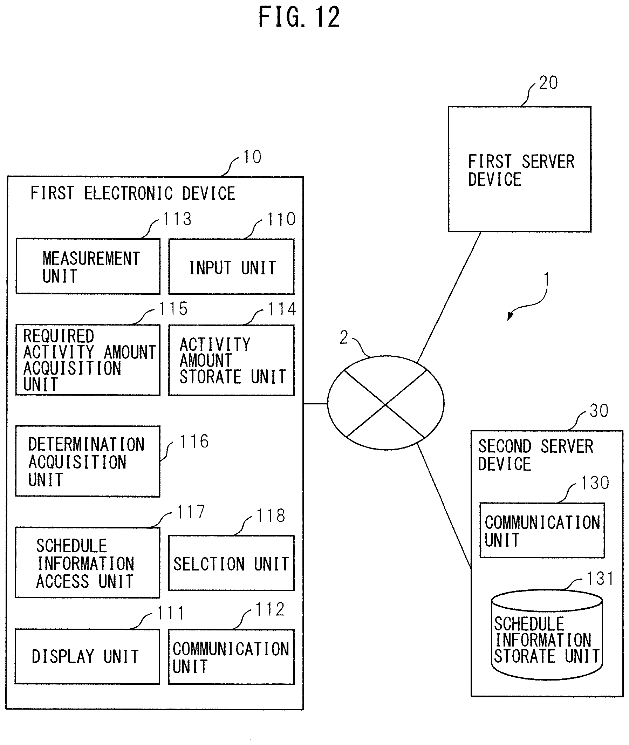

FIG. 12 is a schematic configuration view of the second example of the health preservation system 1. The processor 11 of FIG. 1 carries out information processing with the constituents of the first electronic device 10 depicted in FIG. 12 according to the program stored in the memory 13 and by working in coordination with other hardware elements of the first electronic device 10 as necessary. Further, according to the program stored in the memory 33, the processor 31 of FIG. 11 carries out information processing with the constituents of the second server device 30 depicted in FIG. 12 and by working in coordination with other hardware elements of the second electronic device 30 as necessary. Note that FIG. 12 mainly illustrates functions related to the following descriptions. Thus, the first electronic device 10 and the second server device 30 may include other constituents than the constituents illustrated in FIG. 12. Note that the health preservation system 1 may be realized as a stand-alone computer system. In such a case, for example, the processor 11 of the first electronic device 10 of FIG. 1 may carry out the information processing which is carried out by the first server device 20 and the information processing which is carried out by the second server device 30 as described below by working in coordination with other hardware elements of the first electronic device 10 as necessary.

The second server device 30 includes a communication unit 130 and a schedule information storage unit 131. The communication unit 130 carries out communication processing for exchanging information between the first electronic device 10 and the second server device 30 and between the first server device 20 and the second server device 30. The schedule information storage unit 131 stores the schedule information indicating activity plans of the user of the first electronic device 10.

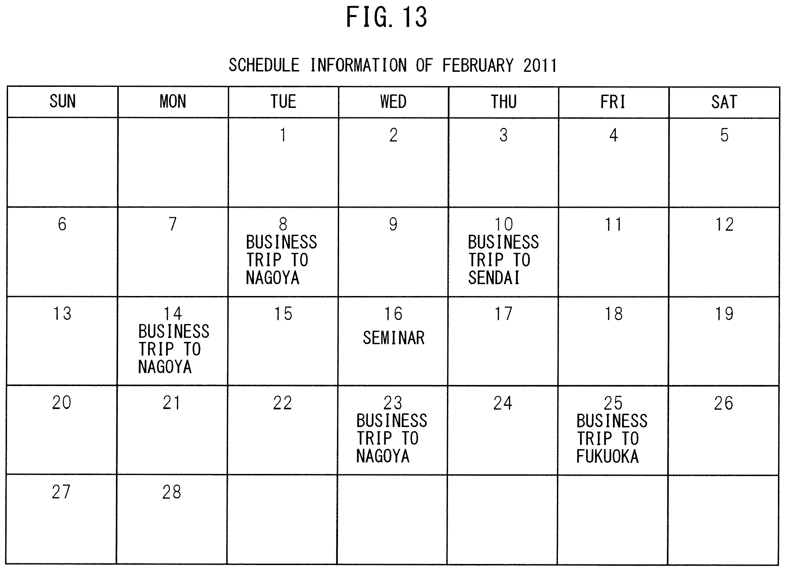

FIG. 13 is an explanatory view of an example of the schedule information. The schedule information of FIG. 13 illustrates activity plans of a user in February 2011. The schedule information indicates that the user goes on business trips on February 8, February 10, February 14, February 23, and February 25, and the user participates in a seminar on February 16. In the following description, each activity plan stored as the schedule information may be referred to as the "event".

Note that while the schedule information illustrated in FIG. 13 designates activity plans of the user in a unit of day, other embodiments may specify activity plans of the user in a unit of other time length. In some embodiments, activity plans of a user may be designated in a unit of hour. In another embodiment, activity plans may be designated in a unit of time such as morning and afternoon, and, in still another embodiment, activity plans may be designated in a unit of week.

Referring to FIG. 12, the first electronic device 10 includes a schedule information access unit 117 and a selection unit 118. The schedule information access unit 117 accesses the schedule information stored in the schedule information storage unit 131 of the second server device 30, and acquires the schedule information of the user. The selection unit 118 selects, based on the acquired schedule information, whether to include an amount of activity measured by the measurement unit 113 during a term corresponding to the schedule information in the amounts of activities which are referred to when calculating the reference amount of activity.

In some embodiments, when some kinds of events, such as "seminar," "business trip," and "party," are designated in the schedule information, the selection unit 118 excludes an amount of activity measured during the term of the event designated in this schedule information from the amounts of activities which are referred to when calculating the reference amount of activity.

In another embodiment, the selection unit 118 determines whether the content of the event designated in the schedule information satisfies a predetermined condition or not. When the designated event satisfies the predetermined condition, the selection unit 118 excludes the amount of activity measured during the term of this event from the amounts of activities which are referred to when calculating the reference amount of activity. The predetermined condition may be set so that the condition is satisfied when the event is a "seminar," "business trip," or "hospitalization," and not satisfied when the event is a "meeting", or the like.

In some embodiments, the term of the amount of activity which the selection unit 118 excludes from the amounts of activities for calculating the reference amount of activity, may be a same term as the term designated by an event. For example, when an event is designated from 6:00 PM to 9:00 PM of December 25, the selection unit 118 may exclude an amount of activity measured during 6:00 PM to 9:00 PM of December 25 from the amounts of activities for calculating the reference amount of activity.

In another embodiment, the selection unit 118 may exclude an amount of activity measured during a term which is of a different length from a term designated by an event from the amounts of activities for calculating the reference amount of activity. For example, the selection unit 118 may exclude an amount of activity which is measured during a term including a term designated by an event, yet has a different length from the term designated by the event from the amounts of activities for calculating the reference amount of activity. For example, when an event is designated from 9:00 AM to 9:30 AM of December 25, the selection unit 118 may exclude an amount of activity measured during a term of one hour represented by a designation of "9:00 AM of December 25" from the amounts of activities for calculating the reference amount of activity. Alternatively, the selection unit 118 may exclude an amount of activity measured during a term of one day represented by a designation of "December 25" from the amounts of activities for calculating the reference amount of activity.

In some embodiments, the selection unit 118 may exclude, from data to be transmitted to the first server device 20, the measured amount of activity which will be excluded from calculation of the reference amount of activity. In another embodiment, the selection unit 118 may inform a term, during which an amount of activity is excluded from calculation of the reference amount of activity, to the reference activity amount acquisition unit 124 of the first server device 20. The reference activity amount acquisition unit 124 calculates the reference amount of activity while excluding the measured amount of activity of the informed term.

In another embodiment, the first electronic device 10 may include the reference activity amount storage unit for calculating the reference amount of activity. In such a case, the selection unit 118 may inform a term during which an amount of activity is excluded from calculation of the reference amount of activity to the reference activity amount acquisition unit. The reference activity amount acquisition unit calculates the reference amount of activity while excluding the measured amount of activity of the informed term. Further, in another embodiment, the selection unit 118 may stop providing the measured amount of activity to the reference activity amount acquisition unit.

In some embodiments, the first electronic device 10 includes a schedule information storage unit which stores schedule information indicating activity plans of the user. The schedule information access unit 117 may access the schedule information stored in the schedule information storage unit provided in the first electronic device 10. In another embodiment, the first server device 20 may carry out the processing of the second server device 30. Further, in another embodiment, the first server device 20 may carry out the processing of the schedule information access unit 117 and the selection unit 118.

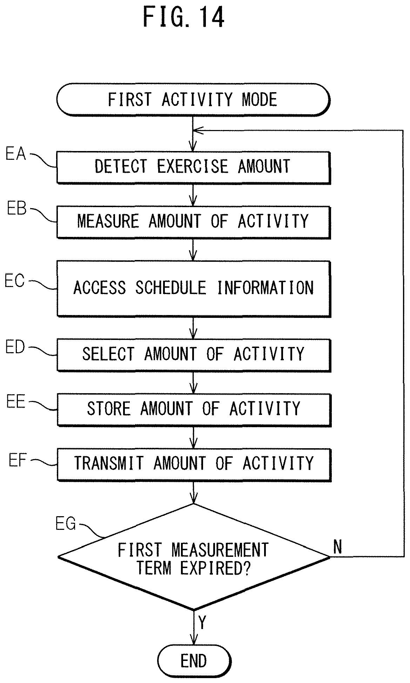

FIG. 14 is an explanatory view of the second example of processing carried out in the first activity mode. Note that in other embodiments, each operation of operations EA to EG may be a step.

The processing of operations EA and EB are the same as those of operations BA and BB depicted in FIG. 4. At operation EC, the schedule information access unit 117 acquires schedule information of a user. At operation ED, the selection unit 118 selects amounts of activities to be excluded from calculation of the reference amount of activity.

At operation EE, the selection unit 118 stores the measured amounts of activities other than the excluded amounts of activities to the activity amount storage unit 114. At operation EF, the first electronic device 10 transmits the amounts of activities stored in the activity amount storage unit 114 to the first server device 20. At operation EG, the first electronic device 10 determines whether the first measurement term has expired. For example, the first electronic device 10 determines whether or not a measurement term other than the terms excluded by the selection unit 118 is longer than a predetermined length defined as the term length of the first measurement term. When the first measurement term has expired (operation EG: Y), the first electronic device 10 terminates the first activity mode. When the first measurement term has not yet expired (operation EG: N), the processing returns to operation EA.

According to this embodiment, since measurement values other than those at normal time are excluded from the measurement values of the amounts of activities as basis for calculating the reference amount of activity, a more appropriate reference amount of activity can be calculated.

Next, another embodiment of the health preservation system 1 will be described. As has been described with reference to FIG. 6, by enabling input of designated amounts of activities by a user, the user is encouraged to do a sport or other exercise which are performed without carrying the first electronic device 10. However, as the user can freely input the designated amounts of activities, correct designated amounts of activities are not necessarily always input.

For this reason, when other people utilize the history data of the amounts of activities collected by the health preservation system 1, credibility of the history data is degraded due to the designated amounts of activities input by the user. The health preservation system 1 of the embodiment described below presents the measured amounts of activities measured by the first electronic device 10 and the designated amounts of activities input by a user in a distinguishable manner to data viewers who will view the history data.

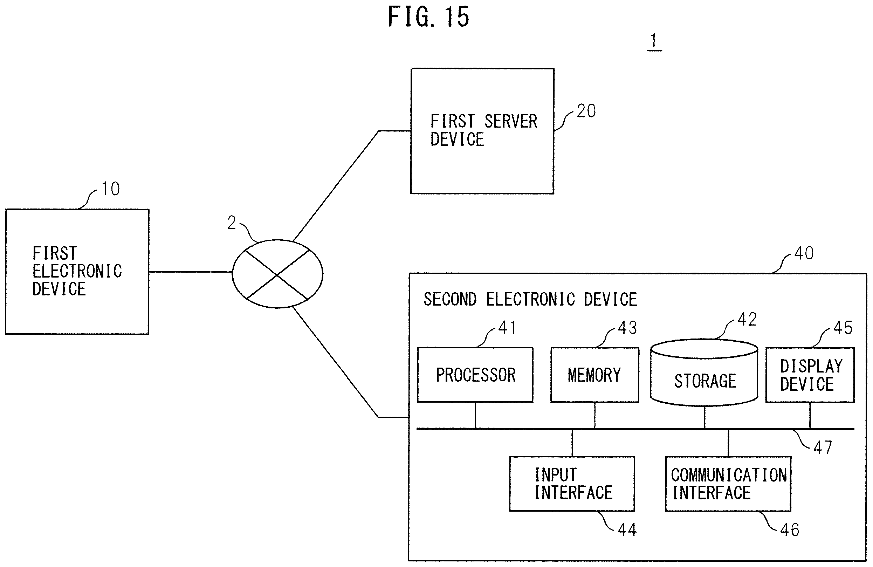

FIG. 15 is a diagram illustrating a hardware configuration of the third example of the health preservation system 1. Constituents equivalent to the constituents illustrated in FIG. 1 are assigned the same reference numerals. The operation of the constituents assigned with the same reference numerals are the same unless otherwise particularly mentioned. Further, the constituents and other functions illustrated in FIG. 15 may also be included in the other embodiments. The health preservation system 1 includes a first electronic device 10, a first server device 20, and a second electronic device 40, which communicate via a communication network 2.

The second electronic device 40 includes a processor 41, a storage 42, a memory 43, an input interface 44, a display device 45, a communication interface 46, and a data bus 47. The second electronic device 30 is an information processing device which carries out an information communication with the first server device 20 via the communication network 2, and can process information to be transmitted to the first server device and information received from the first server device 20.

The second electronic device 40 may be, for example, a mobile telephone, a mobile information terminal, a personal computer, and the like. The second electronic device 40 is used, for example, by a health counselor who views the logs of the amounts of activities and weight of a user of the first electronic device 10 to give health guidance to the user.

The processor 41 carries out processing for controlling operation of the second electronic device 40 by executing a program stored in the storage 42, and information processing according to the executed program. The storage 42 stores the program which causes the processor 41 to execute the above processing. The storage 42 may include a hard disk, a non-volatile memory, or the like as storing means.

The memory 43 stores a program being run by the processor 41 and data which is temporarily used by this program. The memory 43 may include a read-only memory or a random access memory.

The input interface 44 is an input device which accepts input operation by a user. The input interface 44 may be, for example, a keypad, a keyboard, a pointing device, a touch panel, or the like. The display device 45 is a display device which visually displays information processed by the second electronic device 40 to the user. The display device 45 may be, for example, a liquid crystal display, a CRT display, or an organic electroluminescence display. The communication interface 46 carries out transmission and reception processing of signals to and from the first server device 20. The above described constituents 41 to 46 are electrically connected via the data bus 47.

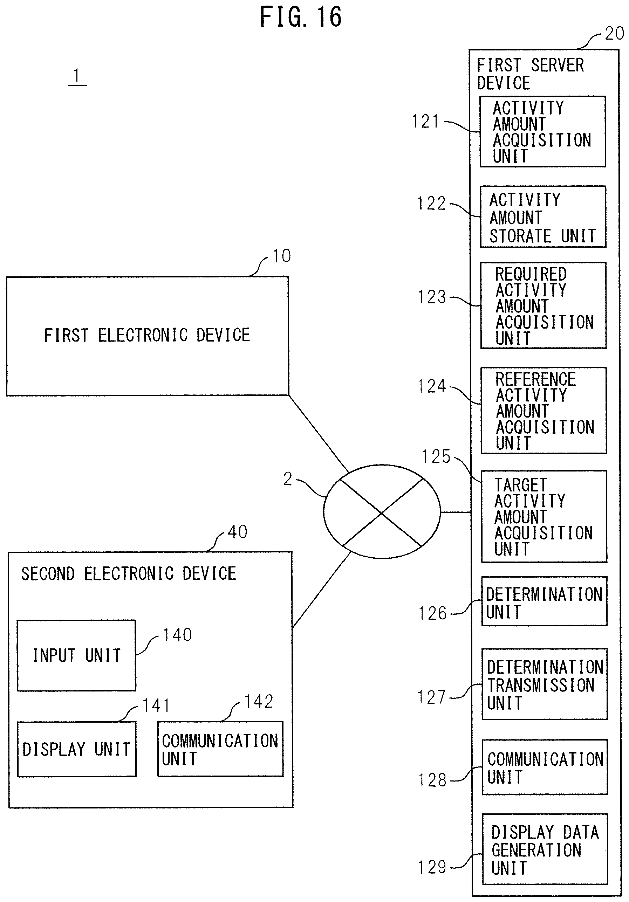

FIG .16 is a schematic configuration view of the third example of the health preservation system 1. The processor 21 of FIG. 1, according to the program stored in the memory 23, carries out information processing with the constituents of the first server device 20 illustrated in FIG. 16 by working in coordination with other hardware elements of the first server device 20 as necessary. Further, the processor 41 of FIG. 15, according to the program stored in the memory 43, carries out information processing with the constituents of the second electronic device 40 illustrated in FIG. 16 by working in coordination with other hardware elements of the second electronic device 40 as necessary. Note that FIG. 16 mainly illustrates functions related to the following description. Thus, the first server device 20 and the second electronic device 40 may include other constituents than the constituents depicted in FIG. 16. Note that the configuration including the first electronic device 10 and the first server device 20 may be realized as a stand-alone computer system. In such a case, for example, the processor 11 of the first electronic device 10 of FIG. 1 may carry out information processing which is executed by the first server device 20 illustrated in FIG. 16 as described below. Further, the processor 11 may carry out information processing which is executed by the second electronic device 40 illustrated in FIG. 16 as described below. In this way, the health preservation system 1 illustrated in FIG. 16 is realized as a stand-alone computer system.

The first server device 20 includes a display data generation unit 129, the generated display data of which will be displayed on the display device 45 of the second electronic device 40. When the second electronic device 40 requests transmission of a history screen for displaying the history data of the amounts of activities of a user, the display data generation unit 129, according to the amounts of activities of the user accumulated in the activity amount storage unit 122, creates the history screen for displaying the amounts of activities of the user and transmits to the second electronic device 40.

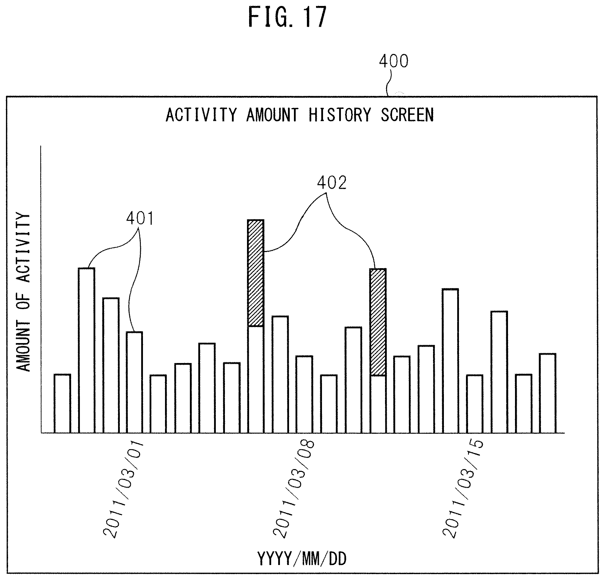

FIG. 17 is a diagram illustrating an example of the activity amount history screen. For example, the activity amount history screen 400 may include a bar chart indicating the amount of activity of the user at each due date. The activity amount storage unit 122 stores measured amounts of activities measured by the measurement unit 113 of the first electronic device 10 and designated amounts of activities input by the user through the input interface 14. The display data generation unit 129 generates the activity amount history screen 400 in a manner in which the measured amounts of activities and the designated amounts of activities are distinguishable. In the example of FIG. 17, the white out graph designated by a reference number 401 indicates a measured amount of activity, and a hatched graph designated by a reference number 402 indicates a designated amount of activity.

Referring back to FIG. 16, the second electronic device 40 includes an input unit 140, a display unit 141, and a communication unit 142. The input unit 140 carries out acceptance processing of a variety of information input by the user via the input interface 44. The display unit 141 displays an input acceptance screen for accepting information input from the input unit 140 on the display device 45. Further, the display unit 141 displays the activity amount history screen 400 received from the first server device 20 on the display device 45. The communication unit 142 carries out communication processing for exchanging information between the second electronic device 20 and the first server device 20.

According to this embodiment, as a user can input a designated amount of activity of exercise which the user carries out without carrying the first electronic device 10, the health preservation system 1 can encourage the user to perform exercise which is difficult to perform while carrying the first electronic device 10. Meanwhile, the health preservation system 1 presents the measured amount of activity and the designated amount of activity in a manner in which the measured amount of activity and the designated amount of activity are distinguishable so that meaningful information can be provided to a data viewer viewing the history data of the amounts of activities even when the viewer does not trust the designated amount of activity input by the user. In this way, the health preservation system 1 of this embodiment can encourage the user to exercise and maintain credibility with the data viewer.

All examples and conditional language recited herein are intended for pedagogical purposes to aid the reader in understanding the invention and the concepts contributed by the inventor to furthering the art, and are to be construed as being without limitation to such specifically recited examples and conditions, nor does the organization of such examples in the specification relate to a showing of the superiority and inferiority of the invention. Although the embodiment(s) of the present inventions have been described in detail, it should be understood that the various changes, substitutions, and alterations could be made hereto without departing from the invention as defined by the claims.

| | |
|---|---|
| 1 | Health Preservation System |
| 10 | First Electronic Device |
| 17 | Sensor |
| 20 | First Server |
| 113 | Measurement Unit |
| 115 | Required Activity amount acquisition Unit |
| 116 | Determination Acquisition Unit |

**Claims**

1. An electronic device (10) comprising:

a measurement unit (113) which is configured to measure an amount of activity of a user according to a signal which indicates an exercise amount and is output from a sensor (17) which detects a physical activity of the user; a required activity amount acquisition unit (115) which is configured to acquire a required amount of activity as the amount of activity for realizing physical characteristics designated by the user, the required amount of activity being calculated by a predetermined calculation formula according to an index value of the designated physical characteristics; a determination acquisition unit (116) which is configured to acquire a determination of a degree of target achievement, with reference to a target amount of activity, achieved by a measured amount of activity which is measured by the measurement unit (113) during a second measurement term after a first measurement term, the target amount of activity being determined by taking a reference amount of activity calculated based on the amount of activity measured by the measurement unit (113) during the first measurement term into consideration with the required amount of activity;

a schedule information access unit (117) which is configured to access schedule information which indicates an activity plan of the user whose amount of activity is to be measured by the measurement unit (113); and

a selection unit (118) which is configured to select based on the schedule information, whether to include the amount of activity measured by the measurement unit (113) during a term corresponding to the schedule information in the amount of activity which is referred to when calculating the reference amount of activity.

2. The electronic device (10) according to claim 1, wherein

the electronic device (10) is configured to selectively operate either in a first state in which the amount of activity of the user is measured by the measurement unit (113) during the first measurement term, or in a second state in which the degree of target achievement is determined.

3. The electronic device (10) according to claim 1 or 2, comprising:

a storage (114) which is configured to store the amount of activity of the user which is measured by the measurement unit (113) during the first term in a manner unable to be accessed through an operation by the user whose amount of activity is measured by the measurement unit (113) or encrypted using an encryption key concealed from the user.

4. The electronic device (10) according to any one of claims 1 to 3, comprising:

an activity amount input unit (110) which is configured to accept input of a designated amount of activity which is the amount of activity designated by the user;

wherein the determination acquisition unit (116) is configured to acquire the determination of the degree of target achievement, with reference to the target amount of activity, which is achieved by the amount of activity obtained by integrating the measured amount of activity measured by the measurement unit (113) and the designated amount of activity accepted by the activity amount input unit (110).

5. A program which causes a computer to execute processes of:

measuring an amount of activity of a user according to a signal which indicates an exercise amount and is output from a sensor which detects a physical activity of the user during a first measurement term and a second measurement term after the first measurement term;

acquiring a required amount of activity as the amount of activity for realizing physical characteristics designated by the user, the required amount of activity being calculated by a predetermined calculation formula according to an index value of the designated physical characteristics;

acquiring a determination of a degree of target achievement, with reference to a target amount of activity, achieved by the amount of activity which is measured during the second measurement term, the target amount of activity being determined by taking a reference amount of activity calculated based on the amount of activity measured by the measurement unit (113) during the first measurement term into consideration with the required amount of activity;

accessing schedule information which indicates an activity plan of the user whose amount of activity is to be measured; and

selecting, based on the schedule information, whether to include the amount of activity measured during a term corresponding to the schedule information in the amount of activity which is referred to when calculating the reference amount of activity.

6. An information system including an electronic device (10) and a server device (20),

the electronic device (10) comprising:

a measurement unit (113) which measures an amount of activity of a user according to a signal which indicates an exercise amount and is output from a sensor which detects a physical activity of the user; and

a transmitting unit (112) which transmits the measured amount of activity measured by the measurement unit to the server device (20);

the server device (20) comprising:

an activity amount acquisition unit (121) which acquires the amount of activity of the user which is measured;

a required activity amount acquisition unit (123) which acquires a required amount of activity as the amount of activity for realizing physical characteristics designated by the user, the required amount of activity being calculated by a predetermined calculation formula according to an index value of the designated physical characteristics;

a reference activity amount acquisition unit (124) which acquires a reference amount of activity which is calculated based on the amount of activity of the user acquired by the activity amount acquisition unit (121) during a first measurement term;

a target activity amount acquisition unit (125) which acquires a target amount of activity which is determined by taking the reference amount of activity into consideration with the required amount of activity;

a determination unit (126) which determines a degree of target achievement, with reference to the target amount of activity, which is achieved by the amount of activity of the user during a second measurement term after the first measurement term;

a schedule information access unit (117) which accesses schedule information which indicates an activity plan of the user whose amount of activity is to be measured by the measurement unit (113); and

a selection unit (118) which selects, based on the schedule information, whether to include the amount of activity measured by the measurement unit (113) during a term corresponding to the schedule information in the amount of activity which is referred to when calculating the reference amount of activity.

7. The information system according to claim 6,
   wherein the electronic device (10) further comprises:

   an activity amount input unit (110) which accepts input of a designated amount of activity as an amount of activity designated by the user; wherein
   the transmission unit (112) transmits the designated amount of activity to the server device (20), and

   wherein the server device (20) further comprises:

   a determination acquisition unit (127) which acquires a determination of a degree of target achievement, with reference to the target amount of activity designated by the user, which is achieved by the amount of activity obtained by integrating the measured amount of activity received from the electronic device (10) and the designated amount of activity; and

   a display data generation unit (129) which generates display data which expresses a change history of the measured amount of activity and the designated amount of activity in a manner in which the measured amount of activity and the designated amount of activity are distinguishable.

8. A health management support method which supports health management of a user, comprising:

   measuring an amount of activity of the user according to a signal which indicates an exercise amount and is output from a sensor which detects a physical activity of the user during a first measurement term and a second measurement term after the first measurement term;

   calculating, using a processor, a required amount of activity as the amount of activity for realizing physical characteristics designated by the user, the required amount of activity being calculated by a predetermined calculation formula according to an index value of the designated physical characteristics;

   accessing, using a schedule information holding unit, schedule information which indicates an activity plan of the user whose amount of activity is to be measured; and

   selecting, using a selecting unit, based on the schedule information, whether to include the amount of activity measured during a term corresponding to the schedule information in the amount of activity which is measured in the first measurement term;

   calculating, using a processor, a reference amount of activity of the user according to the amount of activity measured during the first measurement term, excluding the amount of activity not included in the amount of activity which is measured in the first measurement term;

   calculating, using a processor, a target amount of activity which is determined by taking the reference amount of activity into consideration with the required amount of activity; and

   determining, using a processor, a degree of target achievement, with reference to the target amount of activity, which is achieved by the amount of activity of the user during the second measurement term after the first measurement term.

**Patentansprüche**

1. Elektronische Vorrichtung (10), die Folgendes umfasst:

   eine Messeinheit (113), die konfiguriert ist, um eine Aktivitätsmenge eines Benutzers gemäß einem Signal zu messen, das eine Übungsmenge angibt und von einem Sensor (17) ausgegeben wird, der eine körperliche Aktivität des Benutzers erfasst;
   eine Erfassungseinheit (115) einer erforderlichen Aktivitätsmenge, die konfiguriert ist, um eine erforderliche Aktivitätsmenge als die Aktivitätsmenge zum Verwirklichen physischer Merkmale, die von dem Benutzer festgelegt werden, zu erfassen, wobei die erforderliche Aktivitätsmenge durch eine vorbestimmte Berechnungsformel gemäß einem Indexwert der festgelegten physischen Merkmale berechnet wird;
   eine Bestimmungserfassungseinheit (116), die konfiguriert ist, um eine Bestimmung eines Zielerreichungsgrads bezüglich einer Aktivitätszielmenge zu erfassen, die von einer gemessenen Aktivitätsmenge erzielt wird, die von der Messeinheit (113) während einer zweiten Erfassungszeit nach einer ersten Erfassungszeit gemessen wird, wobei die Aktivitätszielmenge bestimmt wird, indem eine Aktivitätsbezugsmenge, die basierend auf der Aktivitätsmenge berechnet wird, die von der Messeinheit (113) während der ersten Messzeit gemessen wird, mit der erforderlichen Aktivitätsmenge berücksichtigt wird;
   eine Planungsinformationszugriffseinheit (117), die konfiguriert ist, um auf Planungsinformationen zuzugreifen, die eine Aktivitätsplanung des Benutzers, dessen Aktivitätsmenge von der Messeinheit (113) zu messen ist, angibt; und
   eine Auswahleinheit (118), die konfiguriert ist, um basierend auf den Planungsinformationen auszuwählen, ob die Aktivitätsmenge, die von der Messeinheit (113) während einer Zeit gemessen wird, die den Planungsinformationen entspricht, in die Aktivitätsmenge aufzunehmen ist, auf die beim Berechnen der Aktivitätsbezugsmenge Bezug genommen wird.

2. Elektronische Vorrichtung (10) nach Anspruch 1, wobei
   die elektronische Vorrichtung (10) konfiguriert ist, um selektiv entweder in einem ersten Zustand zu arbeiten, in dem die Aktivitätsmenge des Benutzers von der Messeinheit (113) und während der ersten Messzeit gemessen wird, oder in einem zweiten Zustand, in dem der Zielerreichungsgrad bestimmt wird.

3. Elektronische Vorrichtung (10) nach Anspruch 1 oder 2, die Folgendes umfasst:
   einen Speicher (114) der konfiguriert ist, um die Aktivitätsmenge des Benutzers, die von der Messeinheit (113) während der ersten Zeit gemessen wird, auf eine Art zu speichern, die durch einen Vorgang durch den Benutzer, dessen Aktivitätsmenge von der Messeinheit (113) gemessen wird, nicht zugänglich ist, oder unter Verwenden eines Verschlüsselungscodes, der für den Benutzer verborgen ist, verschlüsselt wird.

4. Elektronische Vorrichtung (10) nach einem der Ansprüche 1 bis 3, die Folgendes umfasst:

   eine Aktivitätsmengeneingabeeinheit (110), die konfiguriert ist, um Eingabe einer festgelegten Aktivitätsmenge zu akzeptieren, die die von dem Benutzer festgelegte Aktivitätsmenge ist;
   wobei die Bestimmungserfassungseinheit (116) konfiguriert ist, um die Bestimmung des Zielerreichungsgrads unter Bezugnahme auf die Aktivitätszielmenge, die von der erhaltenen Aktivitätsmenge verwirklicht wird, zu erfassen, indem die gemessene Aktivitätsmenge, die von der Messeinheit (113) gemessen wird, und die festgelegte Aktivitätsmenge, die von der Aktivitätsmengeneingabeeinheit (110) akzeptiert wird, integriert werden.

5. Programm, das einen Computer veranlasst, folgende Prozesse auszuführen:

   Messen einer Aktivitätsmenge eines Benutzers gemäß einem Signal, das eine Übungsmenge angibt und von einem Sensor ausgegeben wird, der eine physische Aktivität des Benutzers während einer ersten Messzeit und einer zweiten Messzeit nach der ersten Messzeit erfasst;
   Erfassen einer erforderlichen Aktivitätsmenge als die Aktivitätsmenge zum Verwirklichen physischer Merkmale, die von dem Benutzer festgelegt werden, wobei die erforderliche Aktivitätsmenge von einer vorbestimmten Berechnungsformel gemäß einem Indexwert der festgelegten physischen Merkmale berechnet wird;
   Erfassen einer Bestimmung eines Zielerreichungsgrads bezüglich einer Aktivitätszielmenge, die von der Aktivitätsmenge erzielt wird, die während der zweiten Messzeit gemessen wird, wobei die Aktivitätszielmenge bestimmt wird, indem eine Aktivitätsbezugsmenge, die basierend auf der Zielmenge, die von der Messeinheit (113) während der ersten Messzeit berechnet wird, mit der erforderlichen Aktivitätsmenge berücksichtigt wird;
   Zugreifen auf Planungsinformationen, die einen Aktivitätsplan des Benutzers, dessen Aktivitätsmenge zu messen ist, angeben; und

basierend auf den Planungsinformationen Auswählen, ob die Aktivitätsmenge, die während einer Zeit gemessen wird, die den Planungsinformationen entspricht, in die Aktivitätsmenge aufzunehmen ist, auf die Bezug genommen wird, wenn die Aktivitätsbezugsmenge berechnet wird.

6. Informationssystem, das eine elektronische Vorrichtung (10) und eine Servervorrichtung (20) umfasst, wobei die elektronische Vorrichtung (10) Folgendes umfasst:

eine Messeinheit (113), die eine Aktivitätsmenge eines Benutzers gemäß einem Signal misst, das eine Übungsmenge angibt und von einem Sensor ausgegeben wird, der eine physische Aktivität des Benutzers erfasst; und eine Übertragungseinheit (112), die die gemessene Aktivitätsmenge, die von der Messeinheit gemessen wird, zu der Servervorrichtung (20) überträgt;

wobei die Servervorrichtung (20) Folgendes umfasst:

eine Aktivitätsmengenerfassungseinheit (121), die die Aktivitätsmenge des Benutzers, die gemessen wird, erfasst;

eine Erfassungseinheit (123) erforderlicher Aktivitätsmenge, die eine erforderliche Aktivitätsmenge als die Menge an Aktivität zum Verwirklichen physischer Merkmale, die von dem Benutzer festgelegt werden, erfasst, wobei die erforderliche Aktivitätsmenge von einer vorbestimmten Berechnungsformel gemäß einem Indexwert der festgelegten physischen Merkmale berechnet wird;

eine Aktivitätsbezugsmengenerfassungseinheit (124), die eine Aktivitätsbezugsmenge erfasst, die basierend auf der Aktivitätsmenge des Benutzers berechnet wird, die von der Aktivitätsmengenerfassungseinheit (121) während einer ersten Messzeit erfasst wird;

eine Aktivitätszielmengenerfassungseinheit (125), die eine Aktivitätszielmenge erfasst, die bestimmt wird, indem die Aktivitätsbezugsmenge mit der erforderlichen Aktivitätsmenge berücksichtigt wird;

eine Bestimmungseinheit (126), die einen Zielerreichungsgrad in Bezug auf die Aktivitätszielmenge bestimmt, die von der Aktivitätsmenge des Benutzers während einer zweiten Messzeit nach der ersten Messzeit verwirklicht wird;

eine Planungsinformationszugriffseinheit (117), die auf Planungsinformationen zugreift, die einen Aktivitätsplan des Benutzers, dessen Aktivitätsmenge von der Messeinheit (113) zu messen ist, angeben; und eine Auswahleinheit (118), die basierend auf den Planungsinformationen auswählt, ob die Aktivitätsmenge, die von der Messeinheit (113) während einer Zeit gemessen wird, die den Planungsinformationen entspricht, in die Aktivitätsmenge aufzunehmen ist, auf die Bezug genommen wird, wenn die Aktivitätsbezugsmenge berechnet wird.

7. Informationssystem nach Anspruch 6, wobei die elektronische Vorrichtung (10) des Weiteren Folgendes umfasst:

eine Aktivitätsmengeneingabeeinheit (110), die Eingabe einer festgelegten Aktivitätsmenge als eine Aktivitätsmenge, die von dem Benutzer festgelegt wird, akzeptiert; wobei die Übertragungseinheit (112) die festgelegte Aktivitätsmenge zu der Servervorrichtung (20) überträgt, und wobei die Servervorrichtung (20) des Weiteren Folgendes umfasst:

eine Bestimmungserfassungseinheit (127), die eine Bestimmung eines Zielerreichungsgrads bezüglich der Aktivitätszielmenge, die von den Benutzer festgelegt wird, erfasst, die von der Aktivitätsmenge verwirklicht wird, die durch Integrieren der gemessenen Aktivitätsmenge, die von der elektronischen Vorrichtung (10) und der festgelegten Aktivitätsmenge erhalten wird; und

eine Anzeigedatenerzeugungseinheit (129), die Anzeigedaten erzeugt, die eine Änderungshistorie der gemessenen Aktivitätsmenge und der festgelegten Aktivitätsmenge auf eine Art ausdrückt, bei der die gemessene Aktivitätsmenge und die festgelegte Aktivitätsmenge unterscheidbar sind.

8. Gesundheitsmanagementunterstützungsverfahren, das das Gesundheitsmanagement eines Benutzers unterstützt, das Folgendes umfasst:

Messen einer Aktivitätsmenge des Benutzers gemäß einem Signal, das eine Übungsmenge angibt und aus einem Sensor ausgegeben wird, der eine physische Aktivität des Benutzers während einer ersten Messzeit und einer zweiten Messzeit nach der ersten Messzeit erfasst;

Berechnen unter Verwenden eines Prozessors einer erforderlichen Aktivitätsmenge als die Aktivitätsmenge zum Verwirklichen physischer Merkmale, die von dem Benutzer festgelegt werden, wobei die erforderliche Aktivitätsmenge durch eine vorbestimmte Berechnungsformel gemäß einem Indexwert der festgelegten physischen Merkmale berechnet wird;

Zugreifen unter Verwenden einer Planungsinformationshalteeinheit auf Planungsinformationen, die einen Aktivitätsplan des Benutzers, dessen Aktivitätsmenge zu messen ist, angeben; und

Auswählen unter Verwenden einer Auswahleinheit, basierend auf den Planungsinformationen, ob die Aktivitätsmenge, die während einer Zeit gemessen wird, die den Planungsinformationen entspricht, in die Aktivitätsmenge, die in der ersten Messzeit gemessen wird, aufzunehmen ist;

Berechnen unter Verwenden eines Prozessors einer Aktivitätsbezugsmenge des Benutzers gemäß der Aktivitätsmenge, die während der ersten Messzeit gemessen wird, ausschließlich der Aktivitätsmenge, die nicht in der Aktivitätsmenge enthalten ist, die in der ersten Messzeit gemessen wird;

Berechnen unter Verwenden eines Prozessors einer Aktivitätszielmenge, die bestimmt wird, indem die Aktivitätsbezugsmenge mit der erforderlichen Aktivitätsmenge berücksichtigt wird; und

Bestimmen unter Verwenden eines Prozessors eines Zielerreichungsgrads mit Bezug auf die Aktivitätszielmenge, die von der Aktivitätsmenge des Benutzers während der zweiten Messzeit nach der ersten Messzeit verwirklicht wird.

## Revendications

1. Dispositif électronique (10) comprenant :

   une unité de mesure (113) qui est configurée de manière à mesurer une quantité d'activité d'un utilisateur selon un signal qui indique une quantité d'exercice et qui est fourni en sortie à partir d'un capteur (17) qui détecte une activité physique de l'utilisateur ;

   une unité d'acquisition de quantité d'activité requise (115) qui est configurée de manière à acquérir une quantité d'activité requise en tant que la quantité d'activité pour atteindre des caractéristiques physiques désignées par l'utilisateur, la quantité d'activité requise étant calculée par une formule de calcul prédéterminée selon une valeur d'index des caractéristiques physiques désignées ;

   une unité d'acquisition de détermination (116) qui est configurée de manière à acquérir une détermination d'un degré de réalisation d'objectif, en référence à une quantité d'activité cible, réalisé par une quantité d'activité mesurée qui est mesurée par l'unité de mesure (113) au cours d'une seconde période de mesure postérieure à une première période de mesure, la quantité d'activité cible étant déterminée en prenant en compte une quantité d'activité de référence calculée sur la base de la quantité d'activité mesurée par l'unité de mesure (113) au cours de la première période de mesure avec la quantité d'activité requise ;

   une unité d'accès à des informations de planification (117) qui est configurée de manière à accéder à des informations de planification qui indiquent un programme d'activité de l'utilisateur dont la quantité d'activité doit être mesurée par l'unité de mesure (113) ; et

   une unité de sélection (118) qui est configurée de manière à sélectionner, sur la base des informations de planification, s'il convient d'inclure la quantité d'activité mesurée par l'unité de mesure (113) au cours d'une période correspondant aux informations de planification, dans la quantité d'activité à laquelle il est fait référence lors du calcul de la quantité d'activité de référence.

2. Dispositif électronique (10) selon la revendication 1, dans lequel :
   le dispositif électronique (10) est configuré de manière à fonctionner sélectivement soit dans un premier état dans lequel la quantité d'activité de l'utilisateur est mesurée par l'unité de mesure (113) au cours de la première période de mesure, soit dans un second état dans lequel le degré de réalisation d'objectif est déterminé.

3. Dispositif électronique (10) selon la revendication 1 ou 2, comprenant :
   un magasin de stockage (114) qui est configurée de manière à stocker la quantité d'activité de l'utilisateur qui est mesurée par l'unité de mesure (113) au cours de la première période, d'une manière inaccessible par le biais d'une opération effectuée par l'utilisateur dont la quantité d'activité est mesurée par l'unité de mesure (113), ou chiffrée en utilisant une clé de chiffrement invisible pour l'utilisateur.

4. Dispositif électronique (10) selon l'une quelconque des revendications 1 à 3, comprenant :

   une unité d'entrée de quantité d'activité (110) qui est configurée de manière à accepter l'entrée d'une quantité d'activité désignée qui correspond à la quantité d'activité désignée par l'utilisateur ;
   dans lequel l'unité d'acquisition de détermination (116) est configurée de manière à acquérir la détermination du degré de réalisation d'objectif, en référence à la quantité d'activité cible, qui est réalisé par la quantité d'activité obtenue, en intégrant la quantité d'activité mesurée qui est mesurée par l'unité de mesure (113) et la quantité d'activité désignée acceptée par l'unité d'entrée de quantité d'activité (110).

5. Programme qui amène un ordinateur à exécuter des processus consistant à :

mesurer une quantité d'activité d'un utilisateur selon un signal qui indique une quantité d'exercice et qui est fourni en sortie à partir d'un capteur qui détecte une activité physique de l'utilisateur, au cours d'une première période de mesure et d'une seconde période de mesure postérieure à la première période de mesure ;
acquérir une quantité d'activité requise en tant que la quantité d'activité pour atteindre des caractéristiques physiques désignées par l'utilisateur, la quantité d'activité requise étant calculée par une formule de calcul prédéterminée selon une valeur d'index des caractéristiques physiques désignées ;
acquérir une détermination d'un degré de réalisation d'objectif, en référence à une quantité d'activité cible, réalisé par la quantité d'activité qui est mesurée au cours de la seconde période de mesure, la quantité d'activité cible étant déterminée en prenant en compte une quantité d'activité de référence calculée sur la base de la quantité d'activité mesurée par l'unité de mesure (113) au cours de la première période de mesure avec la quantité d'activité requise ;
accéder à des informations de planification qui indiquent un programme d'activité de l'utilisateur dont la quantité d'activité doit être mesurée ; et
sélectionner, sur la base des informations de planification, s'il convient d'inclure la quantité d'activité mesurée au cours d'une période correspondant aux informations de planification, dans la quantité d'activité à laquelle il est fait référence lors du calcul de la quantité d'activité de référence.

6. Système d'information incluant un dispositif électronique (10) et un dispositif serveur (20) ;
le dispositif électronique (10) comprenant :

une unité de mesure (113) qui mesure une quantité d'activité d'un utilisateur selon un signal qui indique une quantité d'exercice et qui est fourni en sortie à partir d'un capteur qui détecte une activité physique de l'utilisateur ;
une unité de transmission (112) qui transmet la quantité d'activité mesurée qui est mesurée par l'unité de mesure au dispositif serveur (20) ;
le dispositif serveur (20) comprenant :

une unité d'acquisition de quantité d'activité (121) qui acquiert la quantité d'activité de l'utilisateur qui est mesurée ;
une unité d'acquisition de quantité d'activité requise (123) qui acquiert une quantité d'activité requise en tant que la quantité d'activité pour atteindre des caractéristiques physiques désignées par l'utilisateur, la quantité d'activité requise étant calculée par une formule de calcul prédéterminée selon une valeur d'index des caractéristiques physiques désignées ;
une unité d'acquisition de quantité d'activité de référence (124) qui acquiert une quantité d'activité de référence qui est calculée sur la base de la quantité d'activité de l'utilisateur acquise par l'unité d'acquisition de quantité d'activité (121) au cours d'une première période de mesure ;
une unité d'acquisition de quantité d'activité cible (125) qui acquiert une quantité d'activité cible qui est déterminée en prenant en compte la quantité d'activité de référence avec la quantité d'activité requise ;
une unité de détermination (126) qui détermine un degré de réalisation d'objectif, en référence à la quantité d'activité cible, qui est réalisé par la quantité d'activité de l'utilisateur au cours d'une seconde période de mesure postérieure à la première période de mesure ;
une unité d'accès à des informations de planification (117) qui accède à des informations de planification qui indiquent un programme d'activité de l'utilisateur dont la quantité d'activité doit être mesurée par l'unité de mesure (113) ; et
une unité de sélection (118) qui sélectionne, sur la base des informations de planification, s'il convient d'inclure la quantité d'activité mesurée par l'unité de mesure (113) au cours d'une période correspondant aux informations de planification, dans la quantité d'activité à laquelle il est fait référence lors du calcul de la quantité d'activité de référence.

7. Système d'information selon la revendication 6, dans lequel le dispositif électronique (10) comprend en outre :
une unité d'entrée de quantité d'activité (110) qui accepte l'entrée d'une quantité d'activité désignée en tant qu'une quantité d'activité désignée par l'utilisateur ; dans lequel :

l'unité de transmission (112) transmet la quantité d'activité désignée au dispositif serveur (20) ; et

dans lequel le dispositif serveur (20) comprend en outre :

une unité d'acquisition de détermination (127) qui acquiert une détermination d'un degré de réalisation d'objectif, en référence à la quantité d'activité cible désignée par l'utilisateur, qui est réalisé par la quantité d'activité obtenue en intégrant la quantité d'activité mesurée reçue en provenance du dispositif électronique (10) et la quantité d'activité désignée ; et

une unité de génération de données d'affichage (129) qui génère des données d'affichage qui expriment un historique de changements de la quantité d'activité mesurée et de la quantité d'activité désignée, d'une manière permettant de distinguer la quantité d'activité mesurée et la quantité d'activité désignée.

8. Procédé d'aide à la prise en charge de la santé qui aide à la prise en charge de la santé d'un utilisateur, comprenant les étapes ci-dessous consistant à :

mesurer une quantité d'activité de l'utilisateur selon un signal qui indique une quantité d'exercice et qui est fourni en sortie à partir d'un capteur qui détecte une activité physique de l'utilisateur, au cours d'une première période de mesure et d'une seconde période de mesure postérieure à la première période de mesure ;

calculer, en utilisant un processeur, une quantité d'activité requise en tant que la quantité d'activité pour atteindre des caractéristiques physiques désignées par l'utilisateur, la quantité d'activité requise étant calculée par une formule de calcul prédéterminée selon une valeur d'index des caractéristiques physiques désignées ;

accéder, en utilisant une unité de conservation d'informations de planification, à des informations de planification qui indiquent un programme d'activité de l'utilisateur dont la quantité d'activité doit être mesurée ; et

sélectionner, en utilisant une unité de sélection, sur la base des informations de planification, s'il convient d'inclure la quantité d'activité mesurée au cours d'une période correspondant aux informations de planification, dans la quantité d'activité qui est mesurée au cours de la première période de mesure ;

calculer, en utilisant un processeur, une quantité d'activité de référence de l'utilisateur selon la quantité d'activité mesurée au cours de la première période de mesure, en excluant la quantité d'activité non incluse dans la quantité d'activité qui est mesurée au cours de la première période de mesure ;

calculer, en utilisant un processeur, une quantité d'activité cible qui est déterminée en prenant en compte la quantité d'activité de référence avec la quantité d'activité requise ; et

déterminer, en utilisant un processeur, un degré de réalisation d'objectif, en référence à la quantité d'activité cible, qui est réalisé par la quantité d'activité de l'utilisateur au cours de la seconde période de mesure postérieure à la première période de mesure.

FIG. 1

# FIG. 2

1

**FIRST SERVER DEVICE** 20

121 — ACTIVITY AMOUNT ACQUISITION UNIT

122 — ACTIVITY AMOUNT STORATE UNIT

123 — REQUIRED ACTIVITY AMOUNT ACQUISITION UNIT

124 — REFERENCE ACTIVITY AMOUNT ACQUISITION UNIT

125 — TARGET ACTIVITY AMOUNT ACQUISITION UNIT

126 — DETERMINATION UNIT

127 — DETERMINATION TRANSMISSION UNIT

120 — OMMUNICATION UNIT

**FIRST ELECTRONIC DEVICE** 10

MEASUREMENT UNIT 113

INPUT UNIT 110

REQUIRED ACTIVITY AMOUNT ACQUISITION UNIT 115

ACTIVITY AMOUNT STORAGE UNIT 114

DETERMINATION ACQUISITION UNIT 116

DISPLAY UNIT 111

COMMUNICATION UNIT 112

2

# FIG. 3

```
                    ┌──────────┐
                    │  START   │
                    └────┬─────┘
                         │
         ┌───────────────▼───────────────┐  AA
         │     INPUT SETTING VALUE       │
         └───────────────┬───────────────┘
                         │
         ┌───────────────▼───────────────┐  AB
         │    DETERMINE REQUIRED         │
         │    AMOUNT OF ACTIVITY         │
         └───────────────┬───────────────┘
                         │
         ┌───────────────▼───────────────┐  AC
         ║      FIRST ACTIVITY MODE       ║
         └───────────────┬───────────────┘
                         │
         ┌───────────────▼───────────────┐  AD
         │    DETERMINE REFERENCE        │
         │    AMOUNT OF ACTIVITY         │
         └───────────────┬───────────────┘
                         │
         ┌───────────────▼───────────────┐  AE
         │    DETERMINE TARGET           │
         │    AMOUNT OF ACTIVITY         │
         └───────────────┬───────────────┘
                         │
   AF    ┌───────────────▼───────────────┐
         ║      SECOND ACTIVITY MODE      ║◄──────┐
         └───────────────┬───────────────┘       │
                         │                        │
   AG            ◇────────▼────────◇              │
              ◇                      ◇   N        │
             ◇    STOP USING?         ◇───────────┘
              ◇                      ◇
                 ◇────────┬────────◇
                         │ Y
                    ┌────▼─────┐
                    │   END    │
                    └──────────┘
```

# FIG. 4

AC

$$\boxed{\text{FIRST ACTIVITY MODE}}$$

BA — DETECT EXERCISE AMOUNT

BB — MEASURE AMOUNT OF ACTIVITY

BC — STORE AMOUNT OF ACTIVITY

BD — TRANSMIT AMOUNT OF ACTIVITY

BE — FIRST MEASUREMENT TERM EXPIRED?  N

Y

END

# FIG. 5

AF

```
          ┌──────────────────────────────┐
          │     SECOND ACTIVITY MODE      │
          └──────────────────────────────┘
                        │
CA ──     ┌──────────────────────────────┐
          │     DETECT EXERCISE AMOUNT    │
          └──────────────────────────────┘
                        │
CB ──     ┌──────────────────────────────┐
          │   MEASURE AMOUNT OF ACTIVITY  │
          └──────────────────────────────┘
                        │
CC ──          ╱───────────────────╲        N
              ╱    DETARMINATION     ╲ ───────
              ╲  TIMING HAS COME?    ╱
               ╲───────────────────╱
                        │ Y
          ┌──────────────────────────────┐  CD
          │     DETERMINE DEGREE OF       │
          │     TARGET ACHIEVEMENT        │
          └──────────────────────────────┘
                        │
          ┌──────────────────────────────┐  CE
          │      OUTPUT DEGREE OF         │
          │      TARGET ACHIEVEMENT       │
          └──────────────────────────────┘
                        │
          ┌──────────────┐
          │     END      │
          └──────────────┘
```

# FIG. 6

AF

```
     ┌─────────────────────────────┐
     │    SECOND ACTIVITY MODE      │
     └─────────────────────────────┘
                   │
                   ▼
DA ──  ┌─────────────────────────────┐
       │    DETECT EXERCISE AMOUNT    │
       └─────────────────────────────┘
                   │
                   ▼
DB ──  ┌─────────────────────────────┐
       │   MEASURE AMOUNT OF ACTIVITY │
       └─────────────────────────────┘
                   │
                   ▼
              ╱──────────╲
DC ──       ╱ DESIGNATED  ╲        N
           ╱ AMOUNT OF ACTIVITY╲ ─────►
            ╲   INPUT?         ╱
              ╲──────────╱
                   │ Y
                   ▼
DD ──  ┌─────────────────────────────┐
       │     INPUT DESIGNATED         │
       │     AMOUNT OF ACTIVITY       │
       └─────────────────────────────┘
                   │
                   ▼
              ╱──────────╲
DE ──       ╱ DETERMINATION╲         N
           ╱ TIMING HAS COME?╲ ──────►
            ╲                ╱
              ╲──────────╱
                   │ Y
                   ▼
DF ──  ┌─────────────────────────────┐
       │  INTEGRATE AMOUNT OF ACTIVITY│
       └─────────────────────────────┘
                   │
                   ▼
DG ──  ┌─────────────────────────────┐
       │     DETERMINE DEGREE OF      │
       │     TARGET ACHIEVEMENT       │
       └─────────────────────────────┘
                   │
                   ▼
DH ──  ┌─────────────────────────────┐
       │      OUTPUT DEGREE OF        │
       │      TARGET ACHIEVEMENT      │
       └─────────────────────────────┘
                   │
                   ▼
              ┌─────────┐
              │   END   │
              └─────────┘
```

# FIG. 7

# FIG. 8

300

| | | TOP SCREEN | | |
|---|---|---|---|---|

DATE    [ 2011/××/00 ]    ─310

A. SELECT ACTIVITY TYPE

| A | I | U | E | O |
|---|---|---|---|---|
| KA | KI | KU | KE | KO |
| SA | SHI | SU | SE | SO |
| TA | CHI | TSU | TE | TO |
| NA | NI | NU | NE | NO |
| HA | HI | HU | HE | HO |
| MA | MI | MU | ME | MO |
| YA | | YU | | YO |
| RA | RI | RU | RE | RO |
| WA | | | | |

─311

B. SEARCH ACTIVITY TYPE

─312

[                    ]  [ SEARCH ]  ─313

[          ]

─314

NEW REGISTRATION

# FIG. 9A

320

ACTIVITY TYPE
DESIGNATION SCREEN

○

⦿ SUIÉI (SWIMMING)

○ SUMO

○ SUKASSHU (SQUASH)

○ SUKI-(SKIING)

○ SUKÉ-TO (SKATING)

○

322

321

323 — NEXT

SUNO-BO-DO
(SNOW BOARD)

SUICHU UNDOU
(AQUATIC EXERCISE)

# FIG. 9B

324

ACTIVITY TIME
DESIGNATION SCREEN

325 → SWIMMING (SWIMMING)

326 → ACTIVITY INTENSITY : 8METS

ACTIVITY TIME :

NEXT

327

328

# FIG. 10

330

```
ACTIVITY TYPE
REGISTRATION SCREEN

NEW REGISTRATION IS REQUIRED
AS "BARÉÉ(BALLET)" IS NOT
INCLUDED IN THE LIST OF
ACTIVITY TYPES.
PLEASE ENTER READING AND METS.

NEWLY REGISTERING
ACTIVITY TYPE

┌─────────────────────────┐
│ BARÉÉ(BALLET)           │ ◄──── 331
└─────────────────────────┘


READING

┌─────────────────────────┐
│                         │ ◄──── 332
└─────────────────────────┘

ACTIVITY   ┌──────────────┐
INTENSITY: │              │
           └──────────────┘ ──── 333


  ┌──────────┐      ┌──────────┐
  │  BACK    │      │ SETTING  │
  └──────────┘      └──────────┘
       ▲                 ▲

      334               335
```

# FIG. 11

EP 2 676 605 B1

# FIG. 12

# FIG. 13

SCHEDULE INFORMATION OF FEBRUARY 2011

| SUN | MON | TUE | WED | THU | FRI | SAT |
|---|---|---|---|---|---|---|
| | | 1 | 2 | 3 | 4 | 5 |
| 6 | 7 | 8<br>BUSINESS TRIP TO NAGOYA | 9 | 10<br>BUSINESS TRIP TO SENDAI | 11 | 12 |
| 13 | 14<br>BUSINESS TRIP TO NAGOYA | 15 | 16<br>SEMINAR | 17 | 18 | 19 |
| 20 | 21 | 22 | 23<br>BUSINESS TRIP TO NAGOYA | 24 | 25<br>BUSINESS TRIP TO FUKUOKA | 26 |
| 27 | 28 | | | | | |

EP 2 676 605 B1

# FIG. 14

FIRST ACTIVITY MODE

EA — DETECT EXERCISE AMOUNT

EB — MEASURE AMOUNT OF ACTIVITY

EC — ACCESS SCHEDULE INFORMATION

ED — SELECT AMOUNT OF ACTIVITY

EE — STORE AMOUNT OF ACTIVITY

EF — TRANSMIT AMOUNT OF ACTIVITY

EG — FIRST MEASUREMENT TERM EXPIRED?  N

Y

END

# FIG. 15

<u>1</u>

FIRST SERVER
DEVICE ⌐20

FIRST
ELECTRONIC ⌐10
DEVICE

2⌐✕

⌐40

SECOND ELECTRONIC DEVICE

PROCESSOR ⌐41   MEMORY ⌐43   STORAGE ⌐42   DISPLAY DEVICE ⌐45

⌐47

INPUT INTERFACE   COMMUNICATION INTERFACE

44⌐   46⌐

EP 2 676 605 B1

# FIG. 16

1

FIRST ELECTRONIC DEVICE 10

SECOND ELECTRONIC DEVICE 40

INPUT UNIT 140

DISPLAY UNIT 141

COMMUNICATION UNIT 142

2

FIRST SERVER DEVICE 20

ACTIVITY AMOUNT ACQUISITION UNIT 121

ACTIVITY AMOUNT STORATE UNIT 122

REQUIRED ACTIVITY AMOUNT ACQUISITION UNIT 123

REFERENCE ACTIVITY AMOUNT ACQUISITION UNIT 124

TARGET ACTIVITY AMOUNT ACQUISITION UNIT 125

DETERMINATION UNIT 126

DETERMINATION TRANSMISSION UNIT 127

COMMUNICATION UNIT 128

DISPLAY DATA GENERATION UNIT 129

# FIG. 17

400

ACTIVITY AMOUNT HISTORY SCREEN

AMOUNT OF ACTIVITY

401

402

2011/03/01

2011/03/08

2011/03/15

YYYY/MM/DD

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2005205167 A
- US 20100079291 A1

- WO 2007138822 A1